# EUROPEAN PATENT SPECIFICATION

(11) **EP 1 240 328 B1**
(45) Date of publication and mention of the grant of the patent: **11.01.2006**
(21) Application number: 00977578.4
(22) Date of filing: 24.11.2000
(51) Int. Cl.: C12N 15/31, C07K 14/30, A61K 48/00, A61K 39/02, C12Q 1/68, G01N 33/569, C12N 15/70, C12N 1/21, C07K 16/12

(54) **ANTIGENIC PROTEIN LPPQ OF MYCOPLASMA MYCOIDES SUBSP. MYCOIDES SC., ITS PREPARATION AND USE**
MYCOPLASMA MYCOIDES SUBSP. MYCOIDES SC ANTIGENISCHES LPPQ PROTEIN, SEINE HERSTELLUNG UND VERWENDUNG
PROTEINE LPPQ ANTIGENIQUE DE MYCOIDES SC SOUS-ESPECES DE MYCOPLASMA MYCOIDES, ET PREPARATION ET UTILISATION DE CETTE PROTEINE

(30) Priority: 29.11.1999 EP 99123676
(43) Date of publication of application: 18.09.2002
(73) Proprietor: Akzo Nobel N.V., 6824 BM Arnhem (NL)
(72) Inventor: FREY, Joachim, CH-3012 Bern (CH); NICOLET, Jacques, CH-3074 Bremgarten (CH); ABDO, El-Mostafa, CH-3007 Bern (CH)
(74) Representative: Keus, Jacobus Albertus Ronald
(86) International application number: PCT/EP2000/011798
(87) International publication number: WO 2001/040471

(56) References cited:
- EL-MOSTAFA ABDO ET AL.: "Humoral and bronchial immune responses in cattle experimentally infected with Mycoplasma mycoides subsp. mycoides small colony type" VETERINARY MICROBIOLOGY, vol. 59, no. 2,3, 16 January 1998 (1998-01-16), pages 109-122, XP000982360 cited in the application
- EL-MOSTAFA ABDO ET AL.: "Antigenic and genetic characterization of lipoproteinLppQ from Mycoplasma mycoides subsp. mycoides SC" CLINICAL AND DIAGNOSTIC LABORATORY IMMUNOLOGY, vol. 7, no. 4, July 2000 (2000-07), pages 588-595, XP000982373 & DATABASE EMBL [Online] Entry AF072716, 24 January 2000 (2000-01-24) FREY J. ET AL.: "Mycoplasma mycoides mycoides SC membrane associated lipoprotein precursor" Database accession no. AF072716
- ROSARIO GONCALVES ET AL.: "Antigen heterogeneity among Mycoplasma mycoides subsp. mycoides SC isolates: discrimination of major surface proteins" VETERINARY MICROBIOLOGY, vol. 63, 28 August 1998 (1998-08-28), pages 13-28, XP000982361
- XAOXING CHENG ET AL.: "Characterization of the gene for an immunodominant 72 kDa lipoprotein of Mycoplasma mycoides subsp. mycoides small colony type" MICROBIOLOGY, vol. 142, 1996, pages 3515-3524, XP000982523
- DATABASE EMBL [Online] Entry MCAAW, 19 August 1994 (1994-08-19) BORK P. ET AL.: "M. capricolum DNA for CONTIG MCAAW" Database accession no. Z33359 XP002161528

## Description

The present invention is primarily in the field of veterinary medicine and livestock production. More specifically the invention relates to the protein LppQ of *Mycoplasma mycoides* subsp. *mycoides* SC and DNA encoding said protein. The invention relates to serological detection of *Mycoplasma mycoides* subsp. *mycoides* small colony type (SC) infections, the etiological agent of contagious bovine pleuropneumonia (CBPP) and the design of any type of vaccine, in particular marker vaccines for the differentiation of infected animals from vaccinated animals. The specific lipoprotein LppQ, and especially its N-terminal antigenic part is expressed as recombinant peptide to be used as specific antigen for sero-detection of CBPP.

Contagious bovine pleuropneumonia (CBPP), caused by *Mycoplasma mycoides* subsp. *mycoides* small colony type (SC) is a severe disease affecting cattle as well as buffaloes. CBPP creates great economic losses in endemic regions and is declared by the Office International des Epiozooties (OIE) to be a disease of list "A", which contains the most serious contagious animal diseases; these diseases require special international regulations for their eradication.

CBPP was eradicated from most continents during the 19^{th} century but remained endemic in Africa. In spite of strict sanitary control measures it is re-emerging in Europe since 1980, thus conceiving a serious threat for animal health and for livestock production (ter Laak, 1992; Nicholas and Bashiruddin, 1995; Provost et al., 1987; Egwu et al., 1996).

Serodiagnosis is the most important tool for control of CBPP. Several serological tests have been described during the last fifty years including slide agglutination, complement fixation (CF) (Campbell and Turner, 1953), agar gel precipitation (Gourlay and Shifrine, 1965), enzyme linked immunosorbent assay, passive haemagglutination, Western-blot and dot blot (Nicholas et al., 1996). Complement fixation (CF) test is currently the most widely used serological diagnostic test (Nicholas et al., 1996), and is thought to be specific and sensitive in the acute phase of the disease. However, the CF test is reported to detect only about 70% of chronically infected animals and seems not to detect asymptomatic animals in the early stage of infection (Provost et al., 1987). In addition, problems with specificity of CF test are experienced; recent analysis of samples from monitoring of cattle in an area without CBPP has shown a significant number of false positive results (Stark et al., 1995b; Stark et al., 1995a). Recently a competitive ELISA has been developed for the serodiagnosis of *M. mycoides* subsp. *mycoides* SC infections based on a monoclonal antibody which detects a specific epitope of a 80 kDa antigen of *M. mycoides* subsp. *mycoides* SC (Le and Thiaucourt, 1998). So far no specific antigen of *M. mycoides* subsp. *mycoides* SC and no simple test such as noncompetitive indirect solid phase ELISA (capture of antibodies) currently exists for serodetection of *M. mycoides* subsp. *mycoides* SC infections. For indirect ELISAs, the quality of antigen preparations is decisive for the specificity and the sensitivity of the test. Total cell antigens or extracts of *M. mycoides* subsp. *mycoides* SC contain many antigens which cross react with related mycoplasmas and cannot be used. Part of the present invention comprises the use of a single specific lipoprotein of *M. mycoides* subsp. *mycoides* SC as antigen for serological tests.

Vaccines so far are based on attenuated strains of *M. mycoides* subsp. *mycoides* SC and are used as life vaccines. These vaccines, however, do not permit the differentiation of vaccinated cattle from infected cattle which is a major hinder in control and eradication programmes where vaccination is used.

Recently Abdo et al. (1998) have analysed the course of immune reactions of the manifold antigens of *M. mycoides* subsp. *mycoides* SC in bronchial lavage and serum of cattle experimentally infected with the African strain Afadé (deposited at the European Collection of Cell Cultures, Salisbury, Wiltshire SP4OJG.UK, deposition number: 99081024) and the European strain L2 using Western blots and complement fixation. Several common dominant immunogenic antigens with the molecular masses of 110, 95, 85, 80, 72, 62, 48 and 39 kDa were identified. Since a lot of strains of mycoplasmas very closely related to *M. mycoides* subsp. *mycoides* SC are known, it was questionable if any of the identified immunogenic antigens were specific for *M. mycoides* subsp. *mycoides* SC. However, it was now surprisingly found that the 48 kDa protein listed above has the specificity for *M. mycoides* subsp. *mycoides* SC allowing the person skilled in the art to develop the embodiments of the present invention as described below.

The current invention is the discovery of a lipoprotein antigen named LppQ which is specific to *M. mycoides* subsp. *mycoides* SC and its use or the use of part of this protein, in particular the N-terminal half of the peptide as a specific antigen for serodetection of *M. mycoides* subsp. *mycoides* SC infections by using the peptide e.g. as specific antigen in noncompetitive indirect solid phase ELISA or any other serological test method. The invention includes the gene sequence of *IppQ* for the expression of antigens, or for the use in genetic identification or detection of *M. mycoides* subsp. *mycoides* SC or for the design and production of any type of vaccine against *M. mycoides* subsp. *mycoides* SC. This includes the design of marker vaccines by construction of *M. mycoides* subsp. *mycoides* SC strains which are devoid of LppQ biosynthesis, the formulation of vaccines using LppQ as a component as well as vaccination procedures using the gene sequence of *IppQ* (e.g. DNA vaccines).

The present invention provides a specific protein of the membrane of *M. mycoides* subsp. *mycoides* SC and its corresponding gene and gene sequence data for the specific serodetection of animals infected with *M. mycoides* subsp. *mycoides* SC. The protein, named LppQ, is a lipoprotein of *M. mycoides* subsp. *mycoides* SC. Its corresponding gene *IppQ* was cloned from strain Afade from of *M. mycoides* subsp. *mycoides* SC.

The gene sequence of *IppQ* and the corresponding amino acid sequence of the protein LppQ is presented in Table 5.

The gene *IppQ* is present in the type strain PG1 as well as in all European and African field isolates of *M. mycoides* subsp. *mycoides* SC tested as determined by PCR (see Table 1).

In order to express *IppQ* in recombinant *E. coli* strains and other gene expression systems, the gene sequence was mutated in order to change UGA to UGGₜᵣₚ. In total 9 UGA codons had to be changed to UGGₜᵣₚ by point mutagenesis. Three synthetic genes have been constructed:
- *IppQ** encoding the total *IppQ* gene but containing the universal genetic code that can be read by *E. coli* and other hosts for gene expression (Table 6). The plasmid containing the *IppQ** gene is pJFFmaLP48-MuHis1 (deposited at the European Collection of Cell Cultures, Salisbury, Wiltshire SP40JG.UK, deposition number: 99081025).
- *IppQ*N'* encoding the N'terminal, antigenic, part of LppQ, LppQN'. The *IppQ*N'* contains the universal genetic code (Table 8). The plasmid containing the *IppQ*N'* gene is pJFFLP48-11.
- *IppQ*C',* encoding the C'terminal part of LppQ, LppGZC'. The *IppQ*C'* contains the universal genetic code (Table 10). The plasmid containing the *IppQ*C'* is pJFFmaLppQ-Cterm.

The expression of the three synthetic genes present on the plasmids described above has been achieved using expression vector pETHIS-1. The gene sequence of plasmid pETHIS-1 is available in Genbank/EMBL accession nr AF012911. Plasmid pETHIS1 was constructed to allow the expression of fusion proteins with a N-terminal histidine hexamer and/or a C-terminal histidine decamer in specialised *Escherichia coli* host cells which contain inducible T₇-RNA-polymerase genes as e.g. *E. coli* strain BL21 (DE3) (F-, *ompT*, r-(B),m-(B), lambda DE3, i21,lacl,lacUV5lacZ,T₇-RNA-pol.(lysogenic).int). The polyhistidine tailed LppQ-His peptides were purified by Ni-chelation chromatography.

The N'terminal part of LppQ is the antigenic part of this lipoprotein. This is demonstrated by an immunoblot experiment, the results of which are shown in Table 12. Rabbit antiserum directed against full recombinant LppQ-His recognized both recombinant N-terminal LppQN'-His and recombinant C-terminal LppQC'-His as well as the full LppQ-His protein (Table 12, panel 1). Serum from a cow infected with *M. mycoides* subsp. *mycoides* SC from a CBPP outbreak in Italy from 1992 strongly reacted with the recombinant N-terminal peptide of the protein, LppQN'-His, and with the full LppQ-His, but not with the C-terminal part LppQC'-His (Table 12, panel 2). The same observation was made with serum from an experimental infection. Serum from the early phase (Table 12, panel 4) and from the late phase of the infection showed strong reactions to LppQN' (Table 12, panel 5). No reaction was found in the control serum taken from the animal before infection (Table 12, panel 3).

LppQ, and in particular the N-terminal part of the protein LppQN' is an early and persistent antigenic marker of *M. mycoides* subsp. *mycoides* SC infections. This is demonstrated by immunoblot experiments with sera from controlled experimental infections of cattle with two different strains of *M. mycoides* subsp. *mycoides* SC, an African strain Afade and an European strain L2 (Abdo et al., 1998). The sera from experimentally contact infected animals showed an early, strong and persistent response to LppQN'-His (Table 13). Representative sera from a cow which was contact infected with the European strain L2 showed clear signals on immunoblots containing LppQN'-His starting from day 92 post contact infection, concurrent with the first positive titer of the standard CF test and persisted until day 224 post contact infection (Table 13, part A) when the titer of the standard CF test was below the detection limit for already more than 2 months (Abdo et al., 1998). Representative sera from a cow infected with the African strain Afadé showed a strong signal on day 28 post contact infection, concurrent with the first clear positive CF titer and persisted until day 134 post contact infection (Table 13, part B) when the titer of the standard CF test was below detection limit for 3 weeks (Abdo et al., 1998).

The N-terminal part of LppQ (LppQN') is a specific antigen of *M. mycoides* subsp. *mycoides* SC. Serum from a rabbit that was immunised with total cells of *M. mycoides* subsp. *mycoides* SC type strain PG1 strongly reacted with LppQN' on immunoblots, while sera from rabbits that were immunised with total cells of related mycoplasmas of the *M. mycoides* cluster or *Mycoplasma putrefaciens* showed no reaction with LppQN'-His under the same conditions. In addition sera from Swiss cattle that were positive for *Mycoplasma bovis* but which were free of CBPP showed no reaction with LppQN'-His (Table 14).

The N-terminal part of LppQ (LppQN'-His) is produced in large amounts from plasmid pJFFLP48-11 harboured in *E. coli* strain JF1979 [BL21 (DE3) (F-, *ompT,* r-(B),m-(B), lambda DE3, 121,lacl,lacUV5lacZ,T₇-RNA-pol.(lysogenic),int)/pJFFLP48-11 Ap^{R}]. LppQN'-His is directly purified from guanidium-hydorchloride solubilized cells by Nichelate chromatography. A 50 ml culture of strain JF1979 yielded in 2 mg purified LppQN'-His protein. Other expression systems can be used for the production of LppQN' peptides or derivatives of it.

The invention applies to the production of serological test kits such as immunoblots or noncompetitive indirect solid phase ELISA or any other antibody detection method for *M. mycoides* subsp. *mycoides* SC infections which are useful in screening of animals for CBPP and for carriers of *M. mycoides* subsp. *mycoides* SC.

"Immunological detection systems" means methods to detect antibodies directed against specific components of *M. mycoides* subsp. *mycoides* SC in sera of animals such as the immunoblots (see Table 12) and the ELISA as (see Table 2). These methods will be used for sero-diagnosis of *M. mycoides* subsp. *mycoides* SC infections. Examples for an ELISA test using LppQN'-His-coated microtiterplates are given in Table 2. ELISA based on LppQN'-His-coated Microtiter plates was significantly more sensitive than CF test, in particular with sera taken at late phase post infection (see results Table 2).

The *IppQ* gene can be used to design a system for genetic detection of *M. mycoides* subsp. *mycoides* SC. The specificity of the *IppQ* gene to *M. mycoides* subsp. *mycoides* SC is demonstrated in Table 1 which shows that *IppQ* was present in all *M. mycoides* subsp. *mycoides* SC strains isolated from different continents and countries and at different decades. The *IppQ* gene was not found in other Mycoplasmas in particular not in phenotypically and antigenically closely related animal Mycoplasmas.

In order to explain the invention more clearly, a list of some embodiments of the invention is provided below. This list does not restrict the invention exclusively to these embodiments.
A. One embodiment of the invention is a protein of *Mycoplasma mycoides* subsp. *mycoides* SC comprising the amino acid sequence as shown in table 5 (LppQ precursor, SEQ ID NO: 25), or parts thereof, wherein the parts are preferably characterised by the amino acid sequences as shown in table 7 (mature LppQ, SEQ ID NO: 27), or table 9 (LppQN', SEQ ID NO: 29). Also embodiments of the invention are the LppQ precursor, the mature LppQ protein and the N-terminal half of LppQ (LppQN').
B. Another embodiment of the invention is a protein as described under item A, wherein the sequence of the protein is preceeded and / or followed by 2 to 12 histidine residues, preferably preceeded by six and / or followed by ten histidine residues, respectively.
C. A further embodiment of the invention is a protein as described under item A being a lipoprotein. Another embodiment of the invention is a protein as described under item A being a glycoprotein. Such a glycoprotein may result from expressing a DNA as described under item D in a eukaryotic host cell.
D. Also an embodiment of the invention is a DNA encoding a protein as described under item A, B or C, preferably characterised by a DNA sequence as shown in table 5 (IppQ precursor gene, SEQ ID NO: 24), table 6 (IppQ*, SEQ ID NO: 26), or table 8 (IppQ*N', SEQ ID NO: 28). The DNA may be genomic DNA, cDNA or artificial DNA.
E. An embodiment of the invention is the use of a protein as described under item A, B or C in a diagnostic method for the direct or indirect detection of *Mycoplasma mycoides* subsp. *mycoides* SC, preferably wherein the diagnostic method is an immunological method and is selected from a group comprising immunoblotting, serological tests and ELISA.
F. Another embodiment of the invention is the use of a protein as described under item A, B orC or a DNA as described under item D for the preparation of a vaccine against infection by *Mycoplasma mycoides* subsp. *mycoides* SC; the vaccine itself; and the use of a DNA as described under item D for the preparation of live, attenuated or inactivated cells of *Mycoplasma mycoides* subsp. *mycoides* SC to be used as marker vaccine, wherein said cells are lacking the ability to synthesise the LppQ lipoprotein.
G. Also an embodiment of the invention is a DNA as described under item D in a detection method for *Mycoplasma mycoides* subsp. *mycoides* SC, preferably wherein the detection method is selected from a group comprising PCR and hybridisation methods.
H. A further embodiment of the invention is is the use of a DNA as described under item D for the expression of a protein as described under item A, B or C, a vector comprising said DNA, and a host cell comprising said vector or said DNA.
I. A further embodiment of the invention is a process for the preparation of a protein as described under item A, B or C, wherein
   (a) a vector comprising the DNA as described under item D is introduced into a suitable host cell;
   (b) the host cells are cultivated under conditions allowing the expression of said protein; and
   (c) the protein of step (c) is isolated from the host cells or the supernatant.
J. Also an embodiment of the invention is a process for the preparation of a DNA characterised by the DNA sequence of table 5 (SEQ ID NO: 25), wherein
   (a) a DNA library of genomic DNA of *Mycoplasma mycoides* subsp. *mycoides* SC is cloned into a suitable phage;
   (b) the phage of step (a) is used for infecting suitable host cells;
   (c) the host cells are screened with sera from a mammal infected with *Mycoplasma mycoides* subsp. *mycoides* SC;
   (d) positive clones are selected and purified; and
   (e) the genomic DNA of the phage of step (d) is isolated.
K. A further embodiment of the invention is a process for the preparation of a DNA characterised by the DNA sequence of table 6 (*IppQ**, SEQ ID NO: 26), or table 8 (*IppQ*N'*, SEQ ID NO: 28), wherein
   (a) a DNA characterised by the DNA sequence of table 5 (SEQ ID NO: 24) and suitable sets of primers according to table 3 (SEQ ID NOs: 1 to 4 and 6 to 21) are applied in an overlap extension PCR (OE - PCR) resulting in a first PCR product;
   (b) the first PCR product of the OE - PCR of step (a) is applied in one or more OE-PCR's as required by the number of desired mutations resulting in a final PCR product; and
   (c) the final PCR product is isolated.
L. Also an embodiment of the invention is a process for the detection of antibodies directed to a protein as described under item A, B or C in a body fluid from an animal, wherein
   (a) a protein according to item A, B or C is blotted onto a suitable membrane;
   (b) the membrane is incubated with said body fluid;
   (c) the membrane is incubated with a labelled conjugate; and
   (d) a colour reaction is initiated in order to detect a complex of the antibody of the conjugate and antibodies from the body fluid directed to the protein,
      preferably wherein the body fluid is selected from a group comprising serum and bronchial fluid, the membrane is a nitro-cellulose or nylon membrane and the conjugate is labelled with alkaline phosphatase.
M. Another embodiment of the invention is a process for the detection of antibodies directed to a protein as described under item A, B or C in a body fluid from an animal, wherein
   (a) a microtiter plate is coated with a protein as described under item A, B or C;
   (b) the microtiter plate is incubated with said body fluid;
   (c) the microtiter plate is incubated with a labelled conjugate; and
   (d) a colour reaction is initiated in order to detect a complex of the antibody of the conjugate and antibodies from the body fluid directed to the protein,

   preferably wherein the body fluid is selected from a group comprising serum and bronchial fluid and the conjugate is labelled with alkaline phosphatase.
N. Furthermore, an embodiment of the invention is a process for the detection of *Mycoplasma mycoides* subsp. *mycoides* SC, wherein
   (a) a pair of suitable PCR primers for the detection of a DNA as shown in table 5 (LppQ precursor, SEQ ID NO: 24) is used in a PCR reaction with a sample to be tested; and
   (b) after the PCR the presence of the PCR product to be expected is checked with the suitable pair of PCR primers,

   preferably wherein the suitable pair of PCR primers is selected from a group comprising the primers MMMSCO5-7 (SEQ ID NO: 22) and MMMSCO5-6 (SEQ ID NO: 23) as shown in Table 3.
O. A further embodiment of the invention is a monoclonal antibody specific for a protein according to item A, B or C.
P. Another embodiment of the invention is a diagnostic kit containing a protein according to items A, B or C and / or at least one antibody as according to item O in a suitable container.

Some of the embodiments of the invention as listed above are described in the following more detailed.

The invention includes also LppQ fragments and variants. Polypeptides which qualify as LppQ variants can be produced, pursuant to the present invention, by conventional reverse genetic techniques, i.e., by designing a genetic sequence based upon an amino acid sequence or by conventional genetic splicing techniques. For example, LppQ variants can be produced by techniques which involve site-directed mutagenesis or oligonucleotide-directed mutagenesis. See, for example, "Mutagenesis of Cloned DNA," in CURRENT PROTOCOLS IN MOLECULAR BIOLOGY 8.0.3 et seq. (Ausubel, *et al*. eds. 1989) ("Ausubel").

Other LppQ variants within the present invention are molecules that correspond to a portion of LppQ, or a fragment thereof, or that comprise a portion of LppQ but are not coincident with the natural polypeptide, both of which display the immunogenic activity of LppQ when presented alone or, alternatively, when linked to a carrier. A LppQ variant of this sort could represent an actual fragment of the natural molecule or could be a polypeptide synthesized de novo or recombinantly.

In yet another embodiment, amino acid sequence variants of the protein are prepared. These may, for instance, be minor sequence variants of the protein which arise due to natural variation within the population of that microorganism from which the protein is identified, or they may be homologues of a protein found in other microorganisms. They also may be sequences which do not occur naturally in the protein but which are sufficiently similar that they elicit an immune response when administered to a host. Sequence variants can be prepared by standard methods of site-directed mutagenesis.

Amino acid sequence variants of the protein can be substitutional, insertional or deletion variants. Deletion variants lack one or more residues of the native protein which are not essential for immunogenic activity. Another common type of deletion variant is one lacking secretory signal sequences or signal sequences directing a protein to bind to a particular part of a cell.

Substitutional variants typically contain the exchange of one amino acid for another at one or more sites within the protein, and are designed to modulate one or more properties of the protein such as stability against proteolytic cleavage. Substitutions preferably are conservative, that is, one amino acid is replaced with one of similar shape and charge. Conservative substitutions are well known in the art and include, for example, the changes of: alanine to serine; arginine to lysine; asparigine to glutamine or histidine; aspartate to glutamate; cysteine to serine; glutamine to asparigine; glutamate to aspartate; glycine to proline; histidine to asparigine or glutamine; isoleucine to leucine or valine; leucine to valine or isoleucine; lysine to arginine, glutamine, or glutamate; methionine to leucine or isoleucine; phenylalanine to tyrosine, leucine or methionine; serine to threonine; threonine to serine; tryptophan to tyrosine; tyrosine to tryptophan or phenylalanine; and valine to isoleucine or leucine.

Insertional variants contain fusion proteins such as those used to allow rapid purification of the protein. Other insertional variants can include those in which additional amino acids are introduced within the coding sequence of the protein. These typically are smaller insertions than the fusion proteins described above and are introduced, for example, to disrupt a protease cleavage site.

In another embodiment, major antigenic determinants of the protein are identified by an empirical approach in which portions of the gene encoding the protein are expressed in a recombinant host, and the resulting proteins tested for their ability to protect host animals from challenge. For example, PCR can be used to prepare a range of proteins lacking successively longer fragments of the C-terminus of the protein. The immuno activity of each of these proteins then identifies those fragments or domains of the protein which are essential for this activity. Further experiments in which only a small number of amino acids are removed at each iteration then allows the location of the antigenic determinants of the protein.

Another embodiment for the preparation of the proteins of the invention is the use of peptide mimetics. Mimetics are peptide-containing molecules which mimic elements of protein secondary structure. See, for example, Johnson et al. (1993). The underlying rationale behind the use of peptide mimetics is that the peptide backbone of proteins exists chiefly to orient amino acid side chains in such a way as to facilitate molecular interactions, such as those of antibody and antigen. A peptide mimetic protein of the present invention would, when administered to a host, elicit an immune response which would lead to recognition of the native host cell protein.

Successful applications of the peptide mimetic concept have thus far focussed on mimetics of β-turns within proteins, which are known to be highly antigenic. Likely β-turn structure within an protein of the invention can be predicted by computer-based algorithms as discussed above. Once the component amino acids of the turn are determined, mimetics can be constructed to achieve a similar spatial orientation of the essential elements of the amino acid side chains, as discussed in Johnson *et al., supra.* The mimetic can then be conjugated to a carrier protein for use as a vaccine.

In another embodiment, computer sequence analysis is used to determine the location of the predicted major antigenic determinant epitopes of the recombinant protein. Software capable of carrying out this analysis is readily available commercially, for example MacVector (IBI, New Haven, CT). The software typically uses standard algorithms such as the Kyte/Doolittle or Hopp/Woods methods for locating hydrophilic sequences which are characteristically found on the surface of proteins and are, therefore, likely to act as antigenic determinants. Once this analysis is made, polypeptides can be prepared which contain at least the essential features of the antigenic determinant and which can be employed in vaccines. Genes encoding these determinants can be constructed and inserted into expression vectors by standard methods, for example, using PCR cloning methodology.

As an alternative to recombinant polypeptides, synthetic peptides corresponding to the antigenic determinants can be prepared. Such peptides are at least six amino acid residues long, and may contain up to approximately 35 residues, which is the approximate upper length limit of automated peptide synthesis machines, such as those available from Applied Biosystems (Foster City, CA). Use of such small peptides in vaccines typically requires conjugation of the peptide to an immunogenic carrier protein such as hepatitis B surface antigen. Methods for performing this conjugation are well known in the art.

The invention also relates to a vaccine. In a particularly preferred embodiment, the vaccine comprises the protein as described under item A, B or C or a fragment thereof, in conjunction with a carrier or adjuvant and, where appropriate, auxiliary substances, for immunizing cattle against *Mycoplasma mycoides* subsp. *mycoides* SC.

More generally, however, the vaccine of the present invention is intended for the immunization of a susceptible mammal. Mammals that are susceptible to *Mycoplasma mycoides* subsp. *mycoides* SC in addition to the bovine species include small and large ruminants.

Following purification to an acceptable degree (which is generally in excess of 50% of the total peptide or protein), the antigen can then be administered to animals in the form of a vaccine. In preferred embodiments of the invention, the animals to be immunized are cattle. Where appropriate, whole cells may be used in vaccines. For example Sf9 cells or CHO cells expressing the recombinant antigen could be used directly, in live or killed form, to administer the antigen to host animals. Alternatively live viruses containing nucleic acid sequences encoding the antigens of the invention can be used in vaccines.

The vaccine may include any of a number of different substances referred to as adjuvants, which are known to stimulate the appropriate portion of the immune system of the vaccinated animal. Suitable adjuvants for the vaccination of animals include, but are not limited to oil emulsions such as Freund's complete or incomplete adjuvant (not suitable for livestock use), Marcol 52:Montanide 888 (Marcol is a Trademark of Esso, Montanide is a Trademark of SEPPIC, Paris), squalane or squalene, Adjuvant 65 (containing peanut oil, mannide monooleate and aluminium monostearate), mineral gels such as aluminium hydroxide, aluminium phosphate, calcium phosphate and alum, surfactants such as hexadecylamine, octadecylamine, lysolecithin, dimethyldioctadecylammonium bromide, N,N-dioctadecyl-N,N'-bis(2-hydroxyethyl)-propanediamine, methoxyhexadecylglycerol and pluronic polyols, polyanions such as pyran, dextran sulfate, polyacrylic acid and carbopol, peptides and amino acids such as muramyl dipeptide, dimethylglycine, tuftsin and trehalose dimycolate.

The antigens, expression products and/or synthetic polypeptides of the present invention also can be administered following incorporation into liposomes or other micro-carriers, or after conjugation to polysaccharides, proteins or polymers or in combination with Quil-A to form "Isocoms" (Immunostimulating complexes).

Routes of administration, dosages to be administered, and frequency of injections are all factors which can be optimized using ordinary skill in the art. Typically, the initial vaccination is followed some weeks later by one or more "booster" vaccinations, the net effect of which is the production of vigorous cellular and humoral immune response.

The method of immunizing a mammal against CBPP involves administering to the mammal an effective amount of the foregoing immunogen. Administration may involve any procedure well-known in the art. Any immunization route which may be contemplated or shown to produce an appropriate immune response can be employed, in accordance with the present invention, although subcutaneous administration is preferred. Suitable administration forms include parenteral, intracutaneous or intramuscular injections or preparations suitable for oral, nasal or rectal administration.

The instant invention also relates to antibodies and antisera to natural LppQ, LppQ fragments, recombinant LppQ, or LppQ variants. Such antibodies and antisera are raised using standard vaccination regimes in appropriate hosts. The host is vaccinated with at least one antigen or vaccine of the present invention, with or without an adjuvant, to generate an immune response. The immune response may be monitored, for example, by measurement of the levels of antibodies produced, using standard ELISA methods.

In a preferred embodiment, production of polyclonal antisera is carried out using cattle as host. The host is bled on a regular basis and the antibody fraction purified from the blood by standard means, *e.g*., by protein A or protein G chromatography. In an alternative embodiment, the host is a mouse, and monoclonal antibody-producing hybridomas are prepared by standard means. See CURRENT PROTOCOLS IN IMMUNOLOGY, Coligan *et al*., (1991) ch. 2.5. Monoclonal antibodies are then prepared from the hybridoma cells by standard means.

The invention also relates to a diagnostic kit which contains the protein according to item A, B or C and preferably at least one of the antibodies as described above in suitable containers.

Diagnostic kits for detection of *M. mycoides* subsp. *mycoides* SC infestation are prepared by providing at least one of the antibodies prepared above, together with a positive control standard of a known concentration of the antigen of the present invention. In one embodiment, the antigen positive control is recombinant LppQ or LppQN'. The kit format conforms to a standard diagnostic format such as the ELISA format.

Some aspects of the invention will now be explained in more detail with the aid of the Examples, without being restricted thereto.

### Example 1: Identification of LppQ

In order to investigate the humoral and bronchial immune responses in cattle infected with *M. mycoides* subsp. *mycoides* SC, sequentially collected sera and bronchial washes of cattle which were experimentally contact infected with African strain Afadé or European strain L2 were analysed by immunoblotting (Abdo et al., 1998). Several common dominant immunogenic antigens with the molecular masses of 110, 95, 85, 80, 72, 62, 48 and 39 kDa were identified. The studies revealed - among other immune reactions - an early and persistent immune reaction to a protein of 48 kDa in both sera and bronchial lavages from cattle infected with either strain Afadé, the reference strain for the African cluster of *M. mycoides* subsp. *mycoides* SC (Cheng et al., 1995), or strain L2, the reference strain for the European cluster (Cheng et al., 1995). Now it is shown by immunoblot analysis of antigens from the mycoplasmas which are genetically and antigenically most closely related to *M. mycoides* subsp. *mycoides* SC using the same sera that the 48 kDa was specific to the species *M. mycoides* subsp. *mycoides* SC (Table 1). The 48 kDa antigen is therefore chosen as a candidate for a specific, predominant and persistent antigen for the present development of sero-detection of contagious bovine pleuropneumonia (CBPP) and/or for the development of components of vaccines against CBPP.

### Example 2: Identification and cloning of IppQ gene

The gene *IppQ* encoding the lipoprotein LppQ was cloned by constructing a gene library made with *Sau*3AI digested genomic DNA of *M. mycoides* subsp. *mycoides* SC cloned in λZAP-express™ vector (Stratagene, La Jolla, CA, USA) followed by *in vitro* packaging and infecting compatible *E. coli* strain with the recombinant phages using the "Gigapack gold" packaging kit (Stratagene, La Jolla, CA, USA). The phage plaques were screened with sera from cattle that were experimentally infected with *M. mycoides* subsp. *mycoides* SC strain Afadé (Abdo et al., 1998) using a standard procedure for screening gene libraries (Ausbel et al., 1990). Several phage clones were transformed into phagmid clones using f1 helper phage according to the protocol of the producer of the λZAP-express™ (Stratagene). The DNA sequences of several clones were determined using an ABI Prism model 310 genetic analyser (Applied Biosystems/Perkin-Elmer Cetus, Norwalk, Conn. USA) and the DNA sequences assembly software Sequencher 3.0 (GeneCode, Ann Arbor, MI, USA) to obtain contiguous sequences. One clone, plasmid pJFFma5 showed an ORF encoding a characteristic lipoprotein of 48 kDa as determined by the parameters described (Hayashi 1990). The gene encoding 48 KDa lipoprotein was designated as *IppQ* (Table 5).

### Example 3: Mutagenesis of the 9 codons in the IppQ gene

In order to exchange Mycoplasma specific UGAₜᵣₚ codons to the universal UGGₜᵣₚ codons, site directed mutagenesis of the *IppQ* gene, was performed by the method described by Urban et al. (1997), using oligonucleotide primers listed in Table 3 and the cloned *IppQ* gene on plasmid pJFFma5 as a template. The mutagenised gene with the UGGₜᵣₚ codons is refereed to as *IppQ** (Table 6).

### Example 4: Construction of the plasmids expressing poly-histidine tailed LppQ peptides

Plasmid pJFFmaLP48-MuHis1 expressing poly-histidine tailed IppQ (named LppQ-His) was constructed as follows. The mutagenised *IppQ** gene (Table 6) obtained as described above was used as a template for PCR amplification using oligonucleotide primers P48EcoR1 and P48B7r (Table 3) and Pwo polymerase. The amplified gene was subjected to restriction enzyme digestion using *Eco*R1 and *Bam*H1 and subsequently ligated to the corresponding digested cloning/expression vector pETHIS-1. Plasmid pJFFLP48-11 expressing poly-histidine tailed N'-terminal half of LppQ, named LppQN'-His, was constructed as follows: The mutagenised *IppQ** gene (Table 6) obtained as described above was used as a template for PCR amplification using oligonucleotide primers MMMLP481 and MMMLP484 (Table 3) and Pwo polymerase. The amplified gene fragment *IppQ*N'* (Table 8) was digested with restriction enzymes *Nde*1 and *Bam*H1 and subsequently ligated to the corresponding cloning sites of expression vector pETHIS-1.

Plasmid pJFFmaLppQ-Cterm expressing poly-histidine tailed C-terminal half of LppQ, named LppQC'-His was constructed as follows: the mutagenised *IppQ** gene (Table 6) obtained as described above was used as a template for PCR amplification using oligonucleotide primers P48B1f and P48B7r (Table 3) and *Pwo* polymerase. The amplified gene, named *IppQ*C'* (Table 10) was subjected to restriction enzyme digestion using *Eco*R1 and *Bam*H1 and subsequently ligated to the corresponding cloning sites of *Eco*R1 and *Bam*H1 digested expression vector pETHIS-1.

### Example 5: Expression of the poly-histidine tagged proteins in specialised E. coli

For the expression of the three poly-histidine tailed LppQ-peptides, the *E. coli* strain BL21 (DE3) (Stratagene) was transformed with plasmids pJFFmaLP48-MuHis1, pJFFLP48-11, or pJFFmaLppQ-Cterm (Table 4). Expression and subsequent purification of the poly-histidine tailed peptides was obtained after induction of cell cultures at mid exponential growth phase with IPTG and purification using Ni²⁺-chelation chromatography as described in detail by Braun et al. (1999).

### Example 6: Protocols of Immunoblots

Immunoblots were done as described by Ausubel, et al. (1990). Serum or bronchial lavage fluid were used from the experimental infection as described (Abdo et al., 1998). Purified recombinant proteins (200µg/ml) were mixed with an equal volume of SDS sample buffer (0.06 M Tris HCl pH 6.8, 2% SDS, 10% Glycerol, 2% β-mercaptoethanol, 0.025 % Bromphenol blue), and boiled for 5 min. The antigens were separated by SDS-PAGE polyacrylamide gels using 5-15% gradient polyacrylamide gels, then blotted onto nitro-cellulose membrane with pore size of 0.2 µm (Bio-Rad). The membrane was then blocked with milk buffer for 1 hour at room temperature (r.t.), dried, and cut into strips. The strips were incubated with the sera samples for 1 hour at r.t., and washed 3 times with TBS (100 mM Tris HCl pH 7.5, 150 mM NaCl) for 5 min. The strips were then incubated 1 hour at r.t. with alkaline phosphatase labelled conjugates, which were diluted in milk buffer. The conjugates used were a) Monoclonal antibody (Mab) anti-bovine IgG (Sigma #A7554) diluted 1:5000, b) polyclonal Ab anti-rabbit or anti-mice IgG (Kirkegaard and Perry Gaithersberg, MD, USA) diluted 1:2000. All strips were washed 3 times with TBS buffer. Colour reaction was initiated by 0.3 mg/ml Nitroblue tetrazolium (NBT) (Boehringer Mannheim, Mannheim, Germany), 0.15 mg 5-bromo-4-chloro-3-indolyl phosphate (BCIP) (Boehringer Mannheim) in alkaline buffer, and stopped with distilled water.

### Example 7: Gene IppQ based PCR method as specific system for detection of M. mycoides subsp. mycoides SC

A specific PCR method was developed which allowed the identification of the organism *M. mycoides* subsp. *mycoides* SC based on the *IppQ* gene. The specific primers developed were MMMSCO5-7 and MMMSCO5-6 (Table 3). The specific PCR parameters are: denaturation 94°C, 30 sec.; annealing 48°C, 30 sec.; elongation 72°C, 1 min.; 35 cycles. This PCR amplified a specific 1304 bp fragment from all strains of *M. mycoides* subsp. *mycoides* SC tested which originated of world-wide most distant places (Table 1). No amplification were observed with the other related mycoplasmal organisms (Table 1) under the same conditions.

### Example 8: Immunological detection systems

Purified recombinant LppQN'-His (N-terminal peptide) was produced from strain JF1979 and purified subsequently by Ni-chelation chromatography (Figure 6). Purified LppQN'-His was used to coat Microtiter plates. Coating was done by standard procedures (Nicolet and Martel 1996) using 100µl of LppQ 10µg/ml in carbonate coating buffer (0.1 M Na₂CO₃ /NaHCO₃ pH 9.6). The coated Microtiter plates (containing 1 µg LppQN' per well) were used to perform indirect ELISA on sera from several cattle including cattle with CBPP from different countries, sera from healthy cattle, sera from experimentally infected cattle and sera from healthy cattle including sera which gave false positive results with the standard CF test. The preliminary data from this ELISA are shown in Table 2. The data show that an ELISA test based on LppQN'-His is suitable for specific and sensitive serodiagnosis to detect animals with CBPP or animals that were infected with *M. mycoides* subsp. *mycoides* SC.

### Literature

Abdo, E-M., Nicolet, J., Miserez, R., Goncalves, R., Regalla, J., Griot, C., Bensaide, A., Krampe, M. and Frey, J.: Humoral and bronchial immune responses in cattle experimentally infected with *Mycoplasma mycoides* subsp. *mycoides* small colony type. Vet.Microbiol. 59 (1998) 109-122.

Ausubel, F.M., Brent, R., Kingston, R.E., Moore, D.D., Seidman, J.G., Smith, J.A. and Struhl, K: Current protocols in molecular biology. John Wiley and Sons, Inc. (1990) New York.

Braun, M., Kuhnert, P., Nicolet, J., Burnens, A.P., and Frey, J. (1999) Cloning and characterization of two bistructural S-layer-RTX proteins from *Campylobacter rectus. J.* Bacteriol. 181: 2501-2506.

Campbell, A.D. and Turner, A.W.: Studies on contagious pleuropneumonia of cattle. IV. An improved complement-fixation test. Aust.Vet.J. 29 (1953) 154-163.

Cheng, X., Nicolet, J., Poumarat, F., Regalla, J., Thiaucourt, F., and Frey, J. (1995) Insertion element IS *1296* in *Mycoplasma mycoides* subsp. *mycoides* small colony identifies a European clonal line distinct from African and Australian strains. Microbiology 141:3221-3228.

Coligan *et al*., CURRENT PROTOCOLS IN IMMUNOLOGY, Eds. John Wiley, New York, 1991) ch. 2.5

Egwu, G.O., Nicholas, R.A.J., Ameh, J.A. and Bashiruddin, J.B.: Contagious Bovine Pleuropneumonia: An Update. Vet.Bull. 66 (1996) 875-888.

Gourlay, R.N. and Shifrine, M.: Comparison between methods of antigen preparation and the use of adjuvant in the delayed allergic skin reaction in contagious bovine pleuropneumonia. J.Comp.Pathol. 75 (1965) 375-380.

Hayashi, S, Wu Hc.: Lipoproteins in bacteria: J.Bioenerg. biomembr. 22(3): (1990) 451-471.

Johnson *et al*.,"Peptide Turn Mimetics" in BIOTECHNOLOGY AND PHARMACY, Pezzuto *et al.,* Eds., (Chapman and Hall, New York, 1993)

Le, G.C. and Thiaucourt, F.: A competitive ELISA for the specific diagnosis of contagious bovine pleuropneumonia (CBPP). Vet. Microbiol. 60 (1998) 179-191.

Nicholas, R.A.J. and Bashiruddin, J.B.: *Mycoplasma mycoides* subspecies *mycoides* (small colony variant): The agent of contagious bovine pleuropneumonia and member of the "*Mycoplasma mycoides* cluster". J.Comp.Pathol. 113 (1995) 1-27.

Nicholas, R.A.J., Santini, F.G., Clark, K.M., Palmer, N.M.A., DeSantis, P. and Bashiruddin, J.B.: A comparison of serological tests and gross lung pathology for detecting contagious bovine pleuropneumonia in two groups of Italian cattle. Vet.Rec. 139 (1996) 89-93.

Nicolet J. and Martel, J.L.: ELISA in large animals. In: Molecular and Diagnostic Procedures in Mycoplasmology, Vol. II. J.G: Tully and S. Razin (eds.). (1996) pp 105-113. Academic Press, San Diego, New York.

Provost, A., Perreau, P., Breard, A., Le Goff, C., Martel, J.L. and Cottew, G.S.: Contagious bovine pleuropneumonia. Rev.sci.tech.Off.int.Epiz. 6 (1987) 625-679.

Stark, K.D.C., Vicari, A., Kihm, U. and Nicolet, J.: Surveillance of contagious bovine pleuropneumonia in Switzerland. Rev.sci.tech.Off.int.Epiz. 14 (1995a) 621-629.

Stark, K.D.C., Vicari, A., Tontis, A. and Nicolet, J.: Epidemiological Investigations of Contagious Bovine Pleuropneumonia in Switzerland. Schweiz.Arch.Tierheilkd. 137 (1995b) 92-100.

ter Laak, E.A.: Contagious bovine pleuropneumonia. A review. Vet.Quart. 14 (1992) 104-110.

Urban, A., Neukirchen, S. and Jaeger, K.E: A rapid and efficient method for site-directed mutagenesis using one- step overlap extension PCR. Nucleic Acids Res. 25(11) (1997), 2227-2228.

**Table 1. Strains of mycoplasmas**

| Species* | Strain | Collection* | Origin | Year | Host | *lppQ* |
|---|---|---|---|---|---|---|
| *M. mycoides* subsp. *Mycoides* SC | PG 1 | NCTC | | 1931 | Cattle/type strain | + |
| *M. mycoides* subsp. *Mycoides SC* | 2059 | LPB | Spain | 1984 | Cattle/lung | + |
| *M. mycoides* subsp. *Mycoides SC* | B773/125 | LNV | Portugal | 1991 1 | Cattle/semen | + |
| *M. mycoides* subsp. *Mycoides SC* | C305 | LNV | Portugal | 1993 | Goat/lung | + |
| *M. mycoides* subsp. *Mycoides SC* | 0326 | LNV | Portugal | 1993 | Sheep/milk | + |
| *M. mycoides* subsp. *Mycoides SC* | PO 2 | CIRAD | France | 1980 | Cattle/lung | + |
| *M. mycoides* subsp. *Mycoides SC* | 2022 | LPB | France | 1984 | Cattle/lung | + |
| *M. mycoides* subsp. *Mycoides SC* | L2 | IVBBE | Italy | 1993 | Cattle/lung | + |
| *M. mycoides* subsp. *Mycoides SC* | 402 | LPB | Italy | 1990 | Cattle/lung | + |
| *M. mycoides* subsp. *Mycoides SC* | 6479 | LPB | Italy | 1992 | Cattle/lung | + |
| *M. mycoides* subsp. *Mycoides SC* | Afadé | CIRAD | Chad | 1968 | Cattle | + |
| *M. mycoides* subsp. *Mycoides SC* | Fatick | CIRAD | Senegal | 1968 | Cattle | + |
| *M. mycoides* subsp. *Mycoides SC* | B17 | CIRAD | Chad | 1967 | Zebu | + |
| *M. mycoides* subsp. *Mycoides SC* | 9050-529/1 | CIRAD | Ivory Coast | 1990 | Cattle | + |
| *M. mycoides* subsp. *Mycoides SC* | 91130 | CIRAD | Ctr. Afric. Rep. | 1991 | Cattle | + |
| *M. mycoides* subsp. *Mycoides SC* | 94111 | CIRAD | Rwanda | 1994 | Cattle | + |
| *M. mycoides* subsp. *Mycoides SC* | 95014 | CIRAD | Tanzania | 1995 | Cattle | + |
| *M. mycoides* subsp. *Mycoides SC* | T1/44 | CIRAD | Tanzania | 1952 | Cattle/vaccine strain | + |
| *M. mycoides* subsp. *Mycoides SC* | T1/Sr50 | CIRAD | Tanzania | 1952 | Cattle/vaccine strain | + |
| *M. mycoides* subsp. *Mycoides SC* | KH₃J | CIRAD | Sudan | 1940 | Cattle/vaccine strain | + |
| *M. mycoides* subsp. *Mycoides SC* | Gladysdale | AAHL | Australia | | Cattle | + |
| *M. mycoides* subsp. *Mycoides SC* | V5 | AAHL | Australia | 1965 | Cattle/vaccine strain | + |
| *M. mycoides* subsp. *Mycoides* LC | Y-goat | NCTC | Australia | | Goat/type strain | - |
| *M. mycoides* subsp. *Mycoides* LC | 152/93 | FVLP | Gran Canaria | 1993 | Goat | - |
| *M. mycoides* subsp. *Mycoides* LC | LC8065 | CIRAD | France | | Cattle | - |
| *M. mycoides* subsp. *Mycoides* LC | D2482/91 | IVBBE | Switzerland | | Goat | - |
| *M. mycoides* subsp. *Mycoides* LC | 8794-Inde | CIRAD | India | | Goat | - |
| *M. mycoides*. Subsp. *Capri* | PG3 | NCTC | | | Goat/type strain | - |
| *M. mycoides*. Subsp. *Capri* | N108 | CIRAD | Nigeria | | | - |
| *M. mycoides*. Subsp. *Capri* | capri L | CIRAD | France | | Goat | - |
| *M. mycoides*. Subsp. *Capri* | 9139/11-91 | CIRAD | Turkey | | | - |
| *Mycoplasma* sp. bovine group 7 | PG50 | NCTC | Australia | | Cattle/ref. Strain | - |
| *Mycoplasma* sp. bovine group | B5415 | NVI | Portugal | | Cattle | - |
| *Mycoplasma* sp. bovine group | CP291 | NVI | Portugal | | Goat | - |
| *Mycoplasma* sp. bovine group | FRD424 | BVVG | India | | Goat/milk | - |
| *Mycoplasma* sp. bovine group | Calf 1 | NVI | Nigeria | | | - |
| *Mycoplasma* sp. bovine group | D318b | NVI | Germany | | | - |
| *Mycoplasma* sp. bovine group | C2306 | NVI | Portugal | | | - |
| *Mycoplasma* sp. bovine group | D424 | NVI | Germany | | | - |
| *Mycoplasma* sp. bovine group | QR1/92 | NVI | Australia | | | - |
| *Mycoplasma* sp. bovine group | 4055 | NVI | France | | | - |
| *Mycoplasma* sp. serogroup L | B144P | NVI | USA | | | - |
| *M. capricolum* subsp. *Capricolum* | Calif. Kid | NCTC | California | | Goat/type strain | - |
| *M. capricolum* subsp. *Capricolum* | 173/87 | CIRAD | Greece | | Sheep | - |
| *M. capricolum* subsp. *Capripneumoniae* | F38 | NCTC | Kenya | | Goat/type strain | - |
| *M. capricolum* subsp. *Capripneumoniae* | 9081-487p | CIRAD | Oman | | Goat | - |
| *M. capricolum* subsp. *Capripneumoniae* | Gabès | CIRAD | Tunisia | | Goat | - |
| *M. putrefaciens* | KS1 | NCTC | | | Goat/type strain | - |
| *M. agalactiae* | PG2 | NCTC | | | Goat/type strain | - |
| *M. bovis* | PG45 | NCTC | | | Cattle/type strain | - |

| | | | | | | |
|---|---|---|---|---|---|---|
| *AAHL, Australian Animal Health Laboratory, Geelong, Vic., Australia; BVVG, Jena, Germany; CIRAD, CIRAD-EMVT, Montpellier, France; FVLP, Faculdad de Veterinaria, Universidad de Las Palmas, Spain; IVBBE, Institute for Veterinary Bacteriology, University of Berne, Switzerland; LNV, Laboratorio Nacional de Veterinaria, Lisboa, Portugal; LPB, Laboratoire de Pathologie Bovine; Lyon, France; NCTC, National Collection of Type Cultures, PHLS, London, UK; NVI, National Veterinary Institute, Uppsala, Sweden. | | | | | | |

*IppQ* was determined by PCR and/or by Southern blot hybridization.

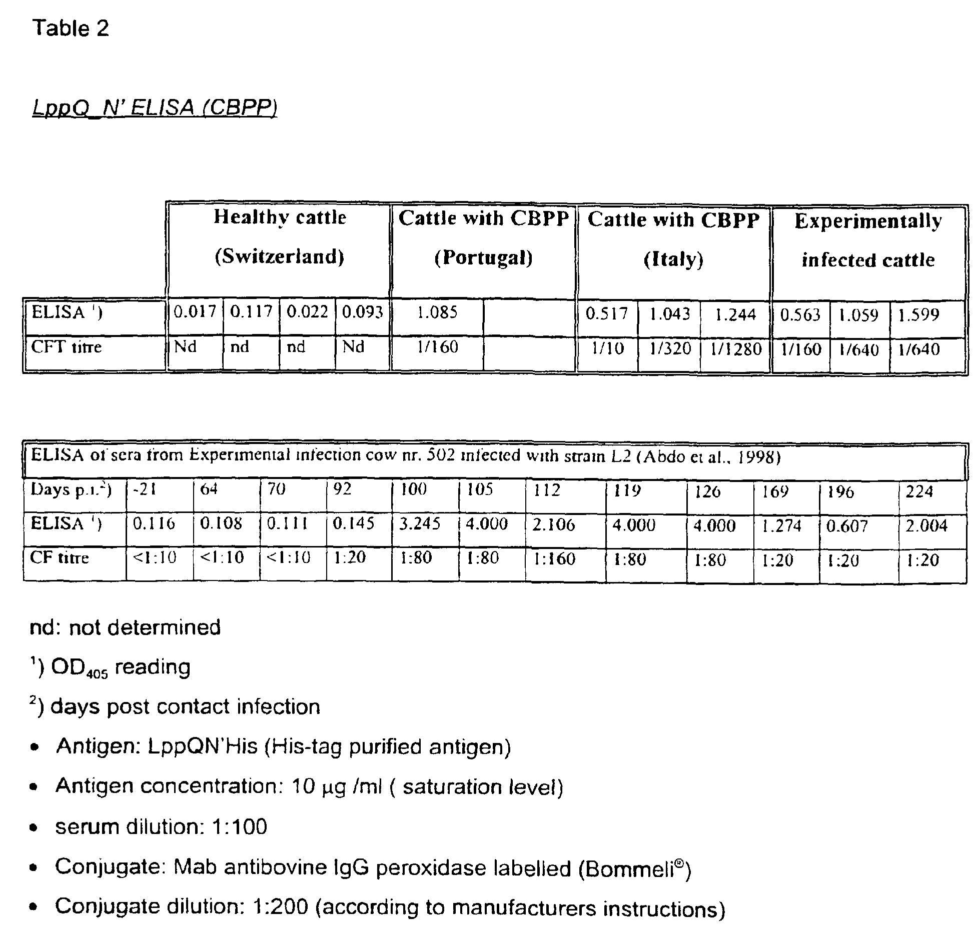

**Table 3**

| Name | Sequence (5' ----- 3') | SEQ ID NO | Temp^{A} | Use ^{B} | Const^{C} |
|---|---|---|---|---|---|
| MMMLP481 | Cgc*catatg*CCATTAGTTGTTGTTTCATGTAAA | 1 | 54°C | SDM, C | 2 |
| MMMLP482 | TCTTGTTTTTGCCACTCATTTTTTG | 2 | 54°C | SDM | |
| MMMLP483 | CAAAAAATGAGTGGCAAAAACAAGA | 3 | 54°C | SDM | |
| MMMLP484 | Cc*ggatcc*CCAATTTGATAAAACTTGATTAAAGT | 4 | 54°C | SDM, C | 2 |
| P48B1f | Aac*gaattc*AGTTTTATCAAATTGGAATACAGAAAAT | 5 | 55°C | SDM, C | 3 |
| P48B1r | CATTTGCTGTTTTCCAGCTCGATAAATC | 6 | 55°C | SDM | |
| P48B2f | GTGATTTATCGAGCTGGAAAACAGCAAATG | 7 | 55°C | SDM | |
| P48B2r | CATTACTTACATTCCAACTCGAAATATCTT | 8 | 55°C | SDM | |
| P48B3f | AAGATATTTCGAGTTGGAATGTAAGTAATG | 9 | 55°C | SDM | |
| P48B3r | ATATTTTTTAGATTCCAATCTGAAAGTGAT | 10 | 55°C | SDM | |
| P48B4f | ATCACTTTCAGATTGGAATCTAAAAAATAT | 11 | 55°C | SDM | |
| P48B4r | CTTTTGATACATCCCAATCTAAAGACTTAT | 12 | 55°C | SDM | |
| P48B5f | ATAAGTCTTTAGATTGGGATGTATCAAAAG | 13 | 55°C | SDM | |
| P48B5r | TTTGAAACATTCCAATTAGTTATATTTTG | 14 | 55°C | SDM | |
| P48B6f | TCAAAATATAACTAATTGGAATGTTTCAAAT | 15 | 55°C | SDM | |
| P48B6r | ACATTATCAACTCTCCAATTTTTTATATCT | 16 | 55°C | SDM | |
| P48B7f | AGATATAAAAAATTGGAGAGTTGATAATGTA | 17 | 55°C | SDM | |
| P48B7r | Cc*ggatcc*TACATTTTTTACATTCCAACTTGAAATATC | 18 | 55°C | SDM, C | 1 & 3 |
| P48FBF | GTTTTATCAAATTGGAATACAGAAAATGTA | 19 | 55°C | SDM | |
| P48FAL | TACATTTTCTGTATTCCAATTTGATAAAAC | 20 | 55°C | SDM | |
| P48 *Eco*RI | Gca*gaattc*CCATTAGTTGTTGTTTCATGTAAA | 21 | 55°C | SDM, C | 1 |
| MMMSCO5-7 | CTCTGAGAGGGAATAATG | 22 | 48°C | D | |
| MMMSCO5-6 | CAACCAAAGGCATCAATG | 23 | 48 | D | |

Lower case *italic* letters indicate the nucleotides that were added in order to create restriction enzyme recognition site (underlined) for cloning
A) annealling temperature for PCR amplification
B) SDM = site directed mutagenesis, C = cloning, D = detection
C) 1= *IppQ** as cloned in pJFFmaLP48-MuHis1
   *2= IppQ*N'* as cloned in pJFFLP48-11
   *3= IppQ*C'* as cloned in pJFFma LppQ-

**Table 4**

| ***Strain*** | Host strain | ***Plasmid*** | *Peptide* |
|---|---|---|---|
| JF2124 | BL21(DE3) | pJFFmaLP48-MuHis1 | LppQ-His |
| JF1979 | BL21 (DE3) | pJFFLP48-11 | LppQN'-His |
| JF2199 | BL21 (DE3) | in pJFFmaLppQ-Cterm | LppQC'-His |
| JF1943 | XL1-blue | pJFFma5 | Non (wild type gene) |

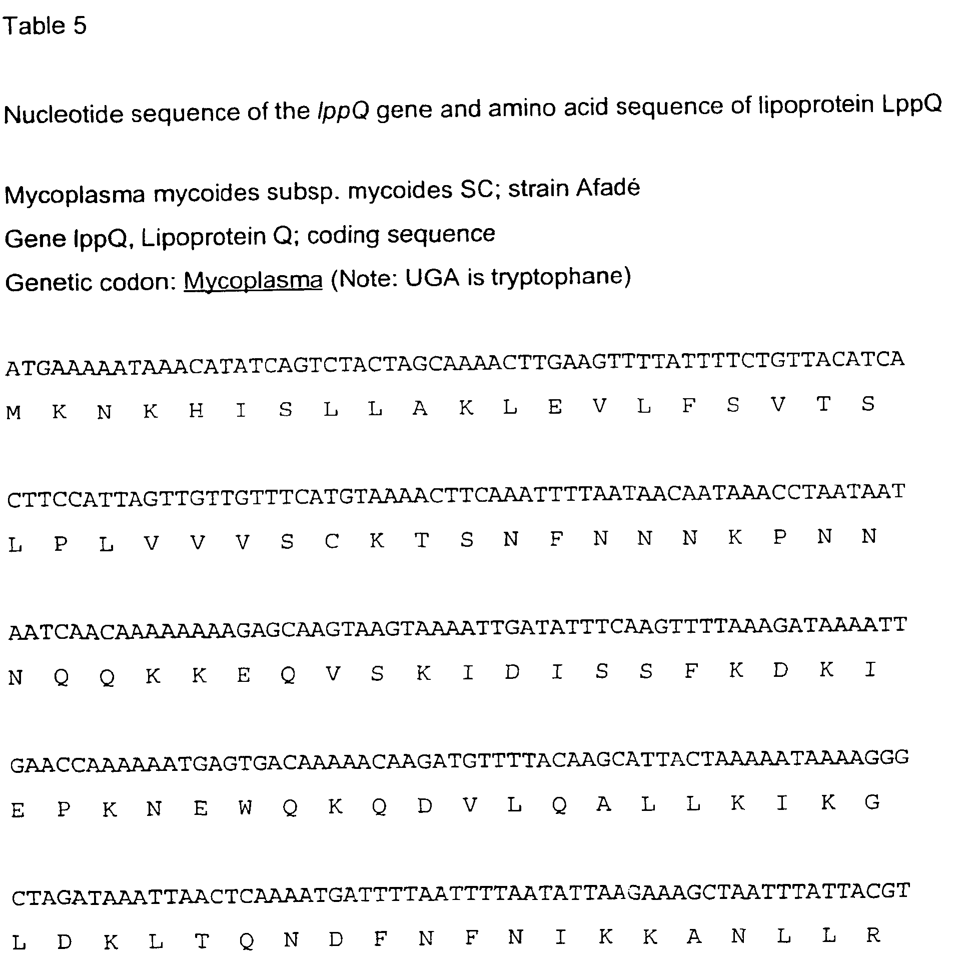

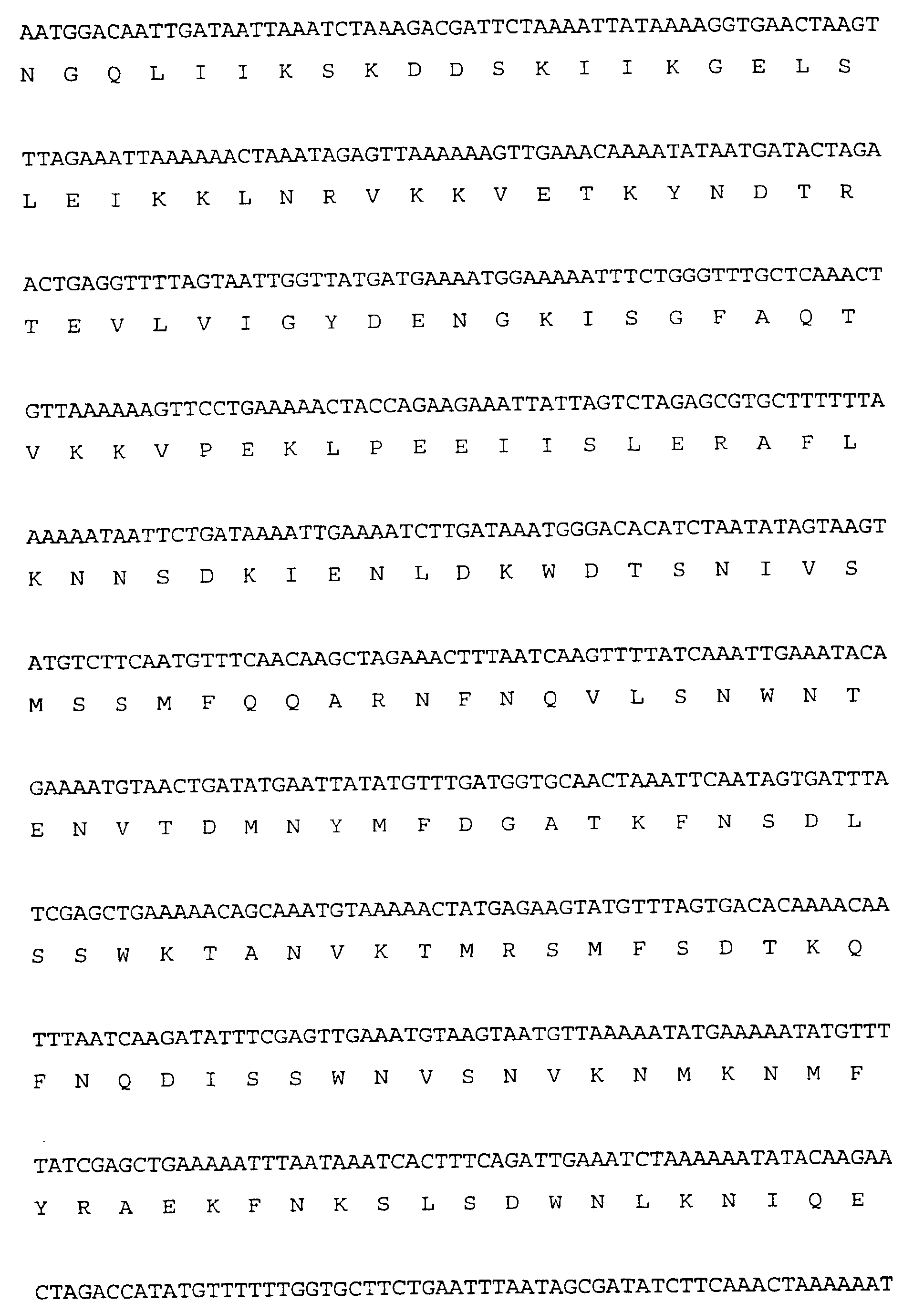

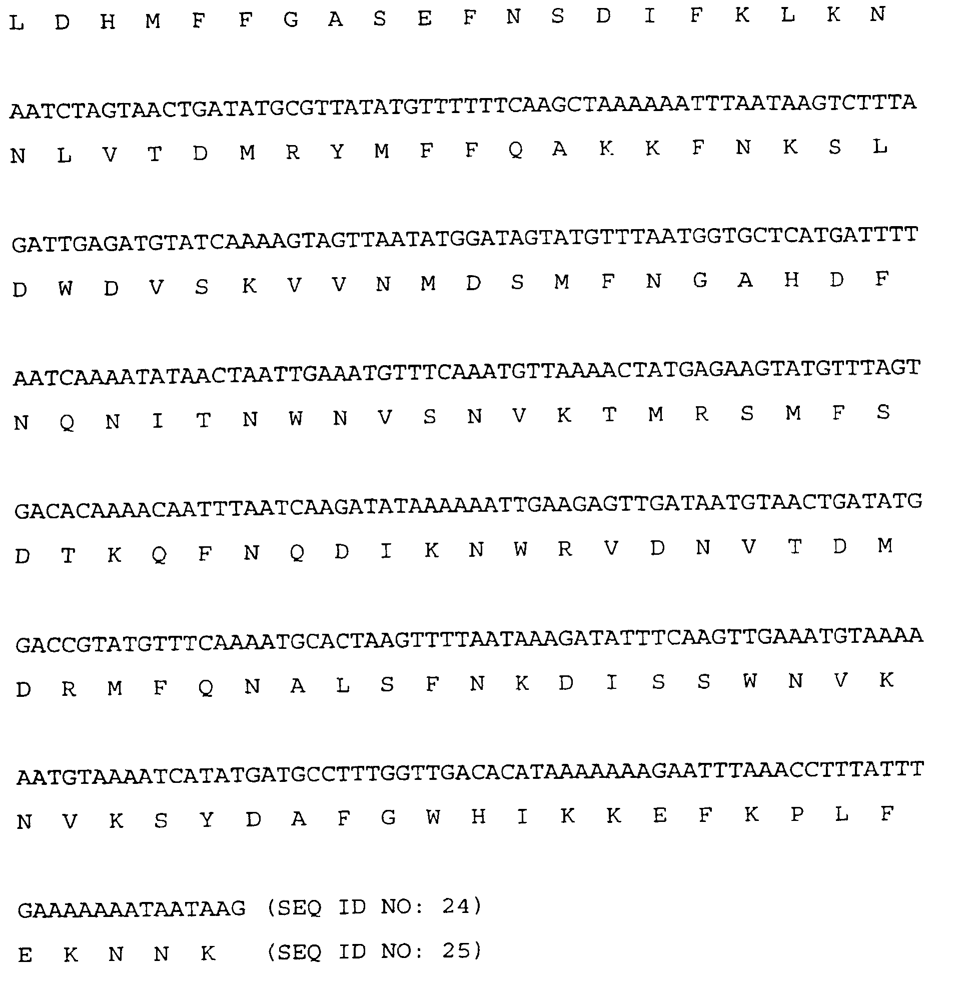

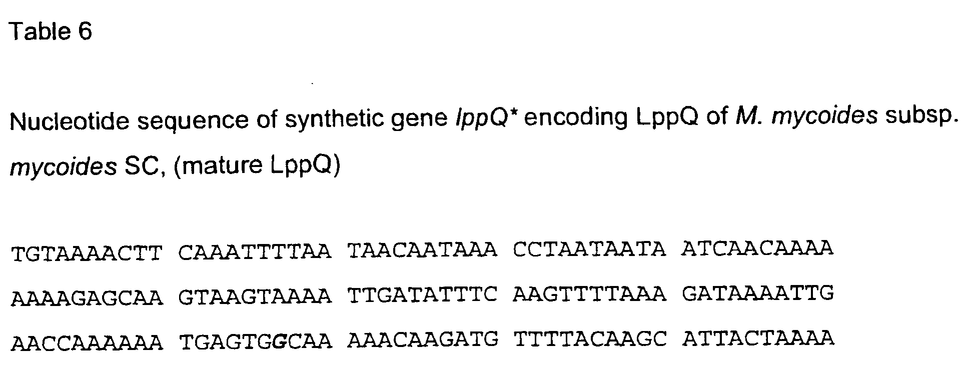

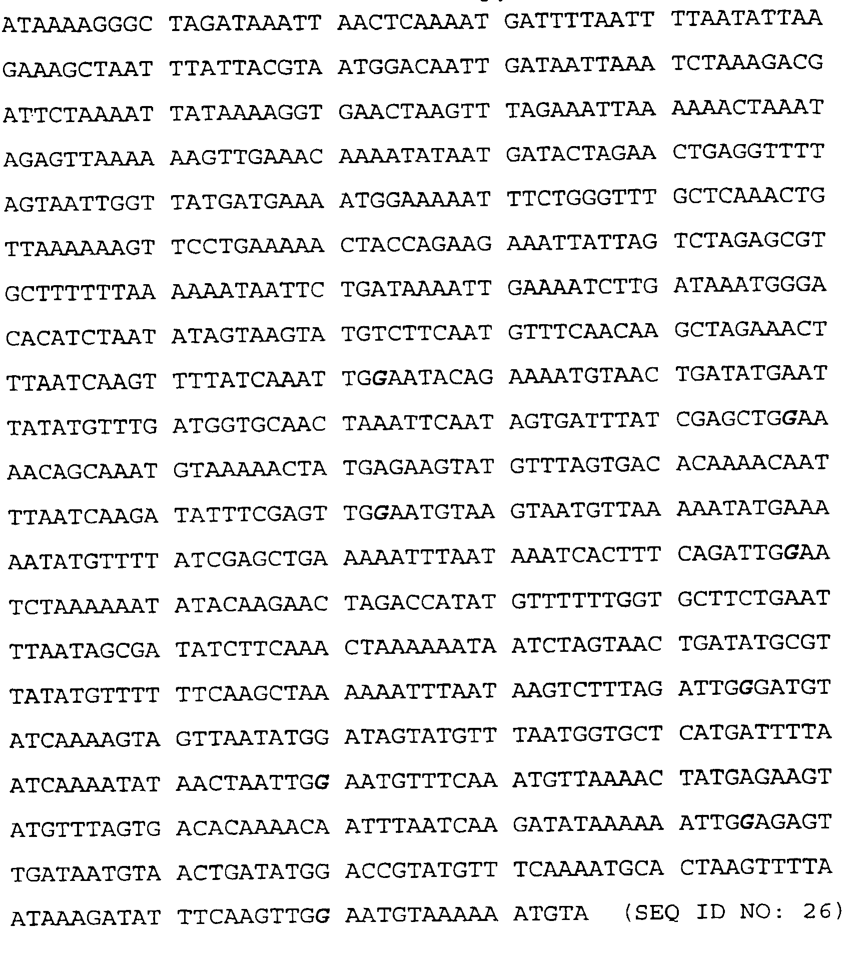

Letters in bold/italic show the point mutations induced in order to obtain the universal UGG_{Trp} codon

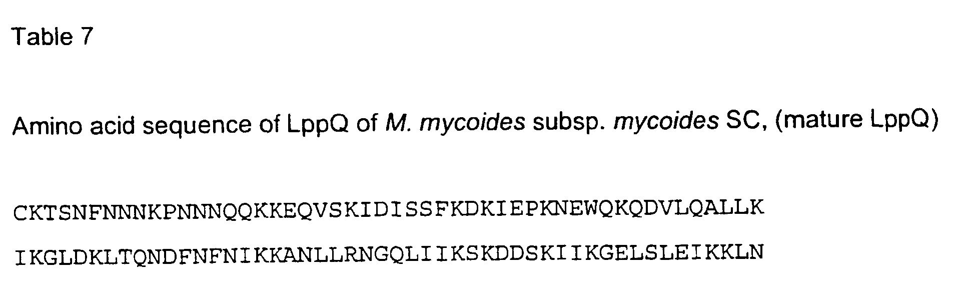

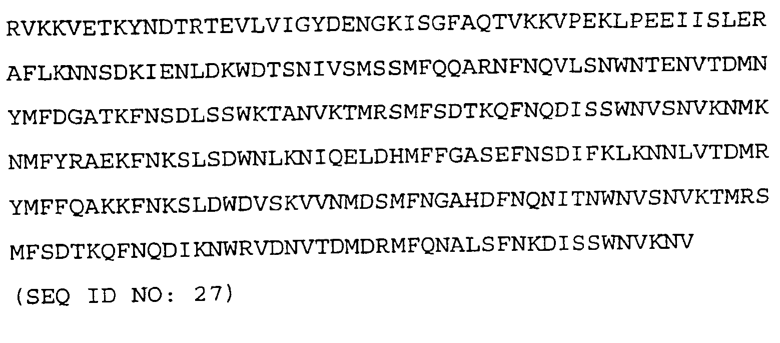

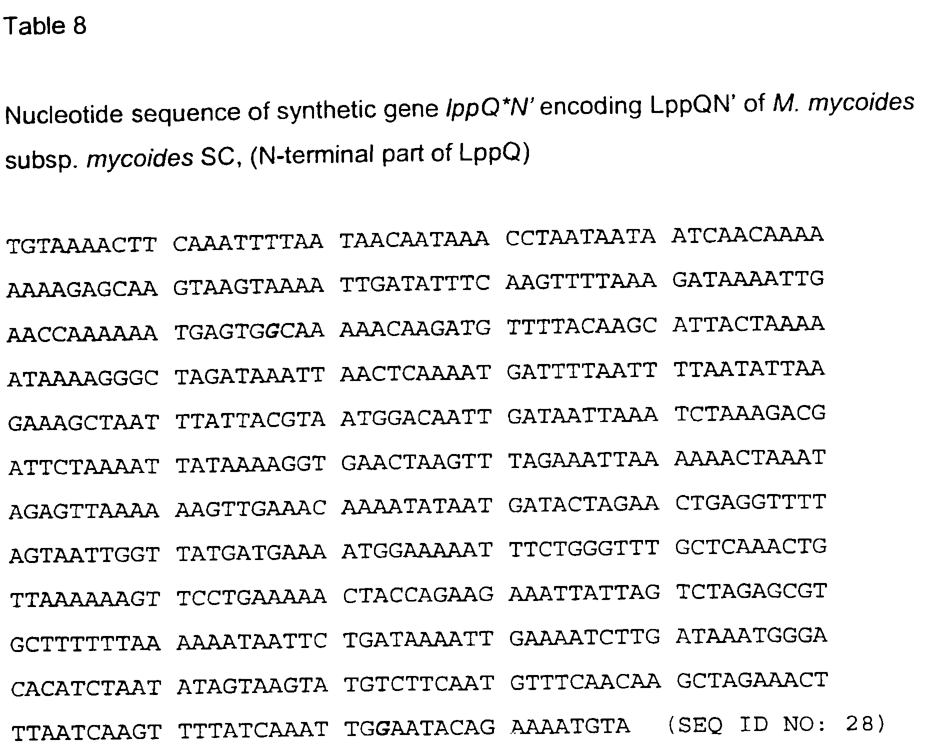

Letters in bold/italic show the point mutations induced in order to obtain the universal UGG_{Trp} codon

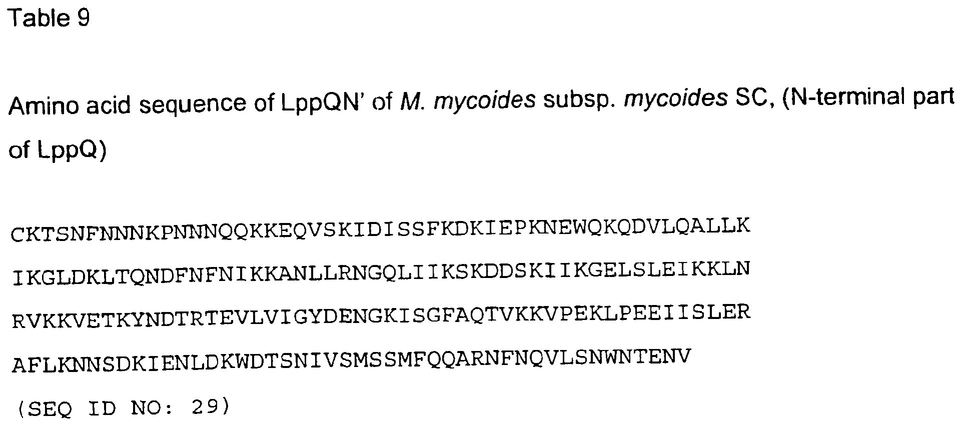

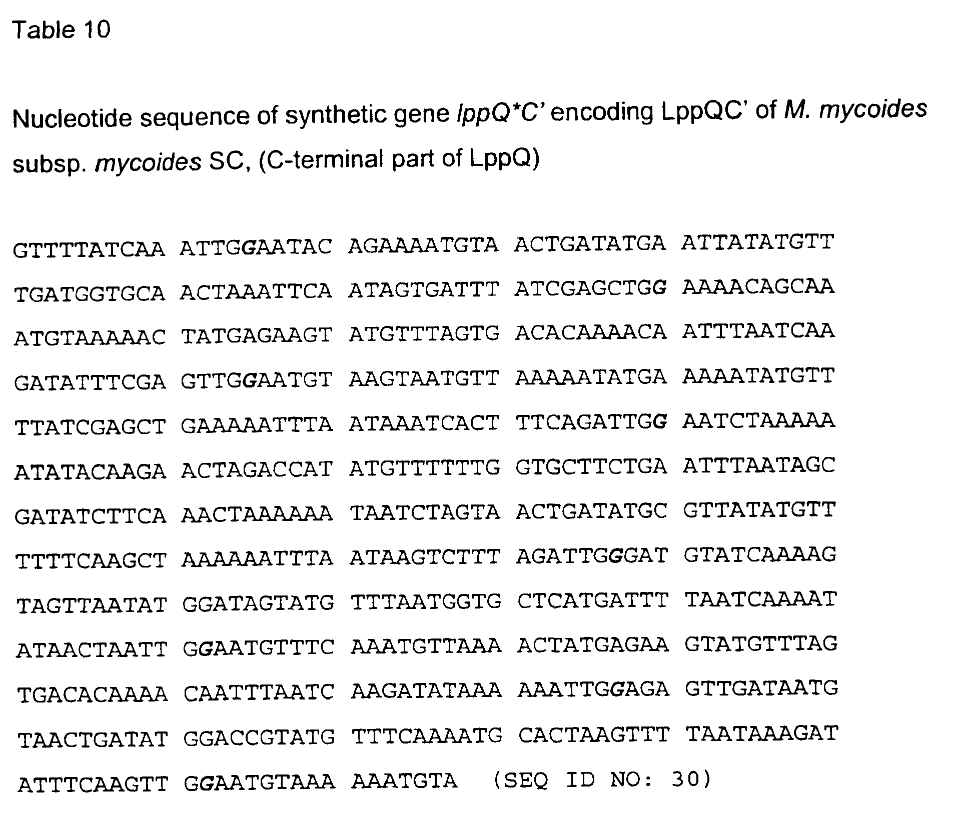

Letters in bold/italic show the point mutations induced in order to obtain the universal UGG_{Trp} codon

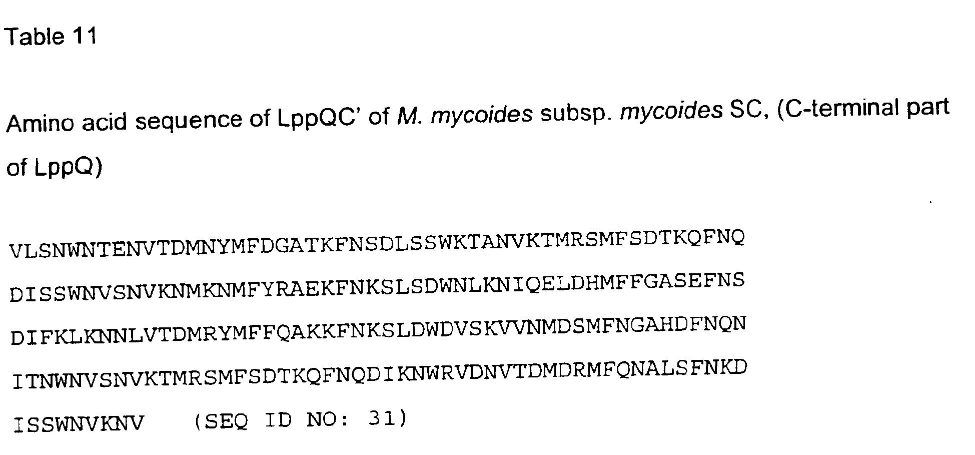

### Table 12

Antigenicity of the N' part and C' part of LppQ from *M. mycoides* subsp. *mycoides* SC

### 1: rabbit anti LppQ-His (1: 1000)

| C | N | F |
|---|---|---|
| + | + | + |

### 2: CBPP cattle serum Italy (1: 100)

| C | N | F |
|---|---|---|
| - | + | + |

### 3: serum cow #511 (1:100) 28 days pre-contact infection*

| C | N | F |
|---|---|---|
| - | - | - |

### 4: serum cow #511 (1: 100) day 28 post contact infection*

| C | N | F |
|---|---|---|
| - | + | + |

### 5: serum cow #511 (1: 100) day 134 post contact infection*

| C | N | F |
|---|---|---|
| - | + | + |

### Antigens:

C: LppQC'-His 1 µg
N: LppQN'-His 1 µg
F: full LppQ-His 0.3 µg

* experimental infection Abdo et al., 1998

### Table 13

Serological reactivity of recombinant LppQN'-His with sequentially collected sera form animals contact-infected with *M. mycoides* subsp. *mycoides* SC strain L2 and Afadé (CBPP)

### Experimental infection with European strain L2

| Days post contact infection | 64 | 70 | 92 | 100 | 105 | 112 | 119 | 126 | 169 | 196 | 224 |
|---|---|---|---|---|---|---|---|---|---|---|---|
| Animal #502 | - | - | + | + | + | + | + | + | + | + | + |

| | | | | | | | | | | | |
|---|---|---|---|---|---|---|---|---|---|---|---|
| note: animal #502 was seronegative before day 92 post infection as determined by CF test (Abdo et al., 1998) | | | | | | | | | | | |

### B. Experimental infection with African strain Afa

| Days post contact infection | -28 | -7 | 7 | 21 | 28 | 35 | 49 | 77 | 98 | 126 | 134 |
|---|---|---|---|---|---|---|---|---|---|---|---|
| Animal #511 | - | - | - | - | + | + | + | + | + | + | + |

| | | | | | | | | | | | |
|---|---|---|---|---|---|---|---|---|---|---|---|
| note: animal #511 CF test was negative on day 21 and before and slightly positive (1/20) on day 28 (Abdo et al., 1998) | | | | | | | | | | | |

Immunoblot conditions: Antigen: 1 µg LppQ/immunoblot
Serum dilution: 1: 100
Method see: Abdo et al., 1998

### Table 14

### Serological specificity of LppQN' to M. mycoides subsp. mycoides SC absence of cross reactions with related animal mycoplasmas

| | 1 | 2 | 3 | 4 | 5 | 6 | 7 | 8 | 9 | 10 | 11 | 12 |
|---|---|---|---|---|---|---|---|---|---|---|---|---|
| LppQN'-His | + | - | - | - | - | - | - | - | - | - | - | - |

### Immunoblots containing 1 µg LppQN'-His were reacted with rabbit hyperimmune serum or with serum from cattle

1: Rabbit serum (1: 1000) against *M. mycoides* subsp. *mycoides* SC (PG 1)
2: Rabbit serum (1: 1000) against *Mycoplasma* sp. bovine gr. 7 (PG50)
3: Rabbit serum (1: 1000) against *M. capricolum* subsp. *capricolum* (Calif kid)
4: Rabbit serum (1: 1000) against *M. mycoides* subsp. *capri* (PG3)
5: Rabbit serum (1: 1000) against *M. capricolum* subsp. *capripneumoniae* (F3 8)
6: Rabbit serum (1: 1000) against *M.* putrifaciens
7: Rabbit serum (1: 1000) against *M. mycoides* subsp. *mycoides* LC (Y-goat)
8: Cattle serum (1:50) positive for *M. bovis* (#68)
9: Cattle serum (1:50) positive for *M. bovis* (#4488/64)
10: Cattle serum (1:50) positive for *M. bovis* (#9/369)
11: Cattle serum (1:50) positive for *M. bovis* (#58/58)

## Claims

1. Isolated Protein of *Mycoplasma mycoides* subsp. *mycoides* small colony (SC) comprising the amino acid sequence as shown in SEQ ID NO: 25, or antigenic parts thereof.

2. Isolated LppQ precursor protein of *Mycoplasma mycoides* subsp. *mycoides* SC **characterized by** the amino acid sequence of SEQ ID NO: 25.

3. Isolated Protein according to claim 1, wherein the parts are **characterized by** the amino acid sequence as shown in SEQ ID NO: 27 or in SEQ ID NO: 29.

4. Isolated Mature LppQ protein of *Mycoplasma mycoides* subsp. *mycoides* SC **characterized by** the amino acid sequence as shown in SEQ ID NO: 27.

5. N-terminal half of mature LppQ protein of *Mycoplasma mycoides* subsp. *mycoides* SC **characterized by** the amino acid sequence as shown in SEQ ID NO: 29.

6. Isolated Protein according to any of claims 1 to 5, wherein the protein is a lipoprotein.

7. DNA encoding a protein according to any of claims 1 to 6.

8. DNA according to claim 7, **characterized by** the DNA sequence as shown in SEQ ID NO: 24, SEQ ID NO: 26, or in SEQ ID NO: 28.

9. Use of a protein according to any of claims 1 to 6 in a diagnostic method for the direct or indirect detection of *Mycoplasma mycoides* subsp. *mycoides* SC.

10. Use according to claim 9, wherein the diagnostic method is an immunological method and is selected from a group comprising immuno-blotting, serological tests and ELISA.

11. Use of a protein according to any of claims 1 to 6 or a DNA according to any of claims 7 or 8 for the preparation of a vaccine against infection caused by *Mycoplasma mycoides* subsp. *mycoides* SC.

12. Use of a DNA according to any of claims 7 or 8 for the preparation of live. attenuated or inactivated cells of *Mycoplasma mycoides* subsp. *mycoides* SC to be used as marker vaccine, wherein said cells are lacking the ability to synthesize the LppQ protein.

13. Use of a DNA according to any of claims 7 or 8 in a detection method for *Mycoplasma mycoides* subsp. *mycoides* SC.

14. Use according to claim 13, wherein the detection method is selected from a group comprising PCR and hybridization methods.

15. Use of a DNA according to any of claims 7 or 8 for the expression of a protein according to any of claims 1 to 6.

16. Vector comprising a DNA according to any of claims 7 or 8.

17. Vector according to claim 16, **characterised in that** said vector is the vector pJFFmaLP48-MuHis1 as deposited at the European Collection of Cell Cultures, Salisbury, Wiltshire SP40JG.UK, under deposition number: 99081025.

18. Host cell comprising a vector according to claim 16 or 17, or a DNA according to any of claims 7 or 8.

19. Process for the preparation of a protein according to any of claims 1 to 6, wherein
(a) a vector according to claim 16 or 17 is introduced into a suitable host cell;
(b) the host cell is cultivated under conditions allowing the expression of a protein according to any of claims 1 to 6; and
(c) the protein of step (b) is isolated from the host cell or the supernatant.

20. Process for the preparation of a DNA **characterized by** the DNA sequence of SEQ ID NO: 25, wherein
(a) a DNA library of genomic DNA of *Mycoplasma mycoides* subsp. *mycoides* SC is cloned into a suitable phage;
(b) the phage of step (a) is used for infecting suitable host cells;
(c) the host cells are screened with sera from a mammal infected with *Mycoplasma mycoides* subsp. *mycoides* SC;
(d) positive clones are selected and purified; and
(e) the genomic DNA of the phage of step (d) is isolated.

21. Process for the preparation of a DNA **characterized by** the DNA sequence of SEQ ID NO: 26, or of SEQ ID NO: 28, wherein
(a) a DNA **characterized in that** it comprises the DNA sequence of SEQ ID NO: 24 and sets of primers according to SEQ ID NOs: 1 to 4 and 6 to 21 are applied in an overlap extension PCR (OE -PCR) resulting in a first PCR product;
(b) the PCR product of the OE -PCR of step (a) is applied in one or more OE PCRs as required by the number of desired mutations resulting in a final PCR product; and
(c) the final PCR product is isolated.

22. Process for the detection of antibodies directed to a protein according to any of claims 1 to 6 in a body fluid from an animal, wherein
(a) a protein according to any of claims 1 to 6 is blotted onto a suitable membrane;
(b) the membrane is incubated with said body fluid;
(c) the membrane is incubated with a labeled conjugate; and
(d) a color reaction is initiated in order to detect a complex of the antibody of the conjugate and antibodies from the body fluid directed to a protein according to any of claims 1 to 6.

23. Process for the detection of antibodies directed to a protein according to any of claims 1 to 6 in a body fluid from an animal, wherein
(a) a microtitre plate is coated with a protein according to any of claims 1 to 6;
(b) the microtitre plate is incubated with said body fluid;
(c) the microtitre plate is incubated with a labeled conjugate; and
(d) a color reaction is initiated in order to detect a complex of the antibody of the conjugate and antibodies from the body fluid directed to a protein according to any of claims 1 to 6.

24. Process for the detection of *Mycoplasma mycoides* subsp. *mycoides* SC, wherein
(a) a pair of PCR primers for the detection of a DNA according to SEQ ID NO: 24 is used in a PCR reaction with a sample to be tested; and
(b) after the PCR reaction the presence of the PCR product to be expected with the pair of PCR primers is checked.

25. Process according to claim 24, wherein the pair of PCR primers is selected from a group comprising the primers of SEQ ID NO: 22 and SEQ ID NO: 23.

26. Monoclonal antibody specific for a protein according to any of claims 1 to 6.

27. Diagnostic kit containing a protein according to any of claims 1 to 6 and/or at least one antibody according to claim 26 in a suitable container.

28. Vaccine comprising a protein according to any of claims 1 to 6 or a DNA according to any of claims 7 or 8 against infection by *Mycoplasma mycoides* subsp. *mycoides* SC.

## Patentansprüche

1. Isoliertes Protein von Mycoplasma mycoides subsp. mycoides SC ("small colony"-Typ) mit der in der SEQ ID Nr. 25 dargestellten Aminosäuresequenz oder antigenische Teile davon.

2. Isoliertes LppQ Precursor Protein von Mycoplasma mycoides subsp. mycoides SC **gekennzeichnet durch** die Aminosäuresequenz nach SEQ ID Nr. 25.

3. Isoliertes Protein nach Anspruch 1, bei dem die Teile durch die Aminosäuresequenz **gekennzeichnet** sind, wie sie in SEQ ID Nr. 27 oder SEQ ID Nr. 29 dargestellt sind.

4. Isoliertes reifes LppQ Protein von Mycoplasma mycoides subsp. mycoides SC, **gekennzeichnet durch** die Aminosäuresequenz, wie sie in SEQ ID Nr. 27 dargestellt ist.

5. N-terminale Hälfte des reifen LppQ Proteins von Mycoplasma mycoides subsp. mycoides SC, **gekennzeichnet durch** die Aminosäuresequenz, wie sie in SEQ ID Nr. 29 dargestellt ist.

6. Isoliertes Protein nach einem der Ansprüche 1 bis 5, bei dem das Protein ein Lipoprotein ist.

7. DNS kodierend ein Protein nach einem der Ansprüche 1 bis 6.

8. DNS nach Anspruch 7, **dadurch gekennzeichnet, dass** die DNS Sequenz wie sie in der Sequenz ID Nr. 24, der Sequenz ID Nr. 26 oder der Sequenz ID Nr. 28 dargestellt ist.

9. Verwendung eines Proteins nach einem der Ansprüche 1 bis 6 in einem Diagnostizierverfahren für die direkte oder indirekte Feststellung von Mycoplasma mycoides subsp. mycoides SC.

10. Verwendung nach Anspruch 9, bei dem das Diagnoseverfahren ein immunologisches Verfahren ist und aus einer Gruppe ausgewählt ist, welche Immuno-Blotting, serologische Tests und ELISA umfasst.

11. Verwendung eines Proteins nach einem der Ansprüche 1 bis 6 oder einer DNS nach einem der Ansprüche 7 oder 8 für die Herstellung eines Impfstoffs gegen die Infektion, die durch Mycoplasma mycoides subsp. mycoides SC hervorgerufen wird.

12. Verwendung einer DNS nach einem der Ansprüche 7 oder 8 für die Herstellung von lebenden attenuierten oder inaktivierten Zellen von Mycoplasma mycoides subsp. mycoides SC, um als Markierungsimpfstoff verwendet zu werden, wobei den besagten Zellen die Fähigkeit zur Synthese des LppQ Proteins fehlt.

13. Verwendung einer DNS nach einem der Ansprüche 7 oder 8 in einem Feststellungsverfahren für Mycoplasma mycoides subsp. mycoides SC.

14. Verwendung nach Anspruch 13, bei dem das Feststellungsverfahren ausgewählt wird aus einem Verfahren umfassend PCR und Hybridisationsverfahren.

15. Verwendung einer DNS nach einem der Ansprüche 7 oder 8 für die Exprimierung eines Proteins nach einem der Ansprüche 1 bis 6.

16. Vektor umfassend eine DNS nach einem der Ansprüche 7 oder 8.

17. Vektor nach Anspruch 16, **dadurch gekennzeichnet, dass** der besagte Vektor der Vektor pJFFmaLP48-MuHis1 ist, wie er bei der European Collection of Cell Cultures, Salisbury, Wiltshire SP40JP.UK unter der Hinterlegungsnummer 99081025 hinterlegt worden ist.

18. Hostzelle mit einem Vektor nach einem der Ansprüche 16 oder 17 oder eine DNS nach einem der Ansprüche 7 oder 8.

19. Verfahren zur Herstellung eines Proteins nach einem der Ansprüche 1 bis 6, wobei
(a) ein Vektor nach Anspruch 16 oder 17 in eine geeignete Hostzelle eingeführt wird,
(b) die Hostzelle unter Bedingungen kultiviert wird, die die Exprimierung eines Proteins nach einem der Ansprüche 1 bis 6 gestatten, und
(c) das Protein nach Schritt (b) aus der Hostzelle oder dem Supernatanten isoliert wird.

20. Verfahren zur Herstellung einer DNS, **gekennzeichnet durch** die DNS Sequenz nach der SEQ ID Nr. 25, wobei
(a) eine DNS Bibliothek von genomischer DNS von Mycoplasma mycoides subsp. mycoides SC in einen geeigneten Phagen geklont wird,
(b) der Phage aus Schritt (a) eingesetzt wird, um geeignete Hostzellen zu infizieren,
(c) die Hostzellen mit Seren gescreent werden, die aus einem Säugetier stammen, welches mit Mycoplasma mycoides subsp. mycoides SC infiziert ist,
(d) positive Klone ausgewählt und gereinigt werden, und
(e) die genomische DNS des Phagen aus Schritt (d) isoliert wird.

21. Verfahren zur Herstellung einer DNS, **gekennzeichnet durch** die DNS Sequenz nach SEQ ID Nr. 26 oder SEQ ID Nr. 28, wobei
(a) eine DNS **dadurch gekennzeichnet ist**, dass sie die DNS Sequenz der SEQ ID Nr. 24 enthält, und Sätze von Primern nach SEQ ID's Nr. 1 bis 4 und 6 bis 21 in einer überlappenden Erweiterung von PCR (OE-PCR) angewandt werden, was in einem ersten PCR-Produkt resultiert,
(b) das PCR-Produkt nach der OE-PCR des Schrittes (a) auf eine oder mehrere OE-PCRs angewandt wird, wie es erforderlich ist aufgrund der Anzahl der gewünschten Mutationen, die in einem endgültigen PCR-Produkt resultieren, und
(c) das endgültige PCR-Produkt isoliert wird.

22. Verfahren zur Erfassung von Antikörpern, die auf ein Protein nach einem der Ansprüche 1 bis 6 ausgerichtet sind, in einer Körperflüssigkeit eines Tieres, wobei
(a) ein Protein nach einem der Ansprüche 1 bis 6 in eine geeignete Membran geblottet wird,
(b) die Membran mit der besagten Körperflüssigkeit inkubiert wird,
(c) die Membran mit einem markierten Konjugat inkubiert wird, und
(d) eine Farbreaktion initiiert wird, um einen Komplex des Antikörpers des Konjugats und Antikörper aus der Körperflüssigkeit, die auf ein Protein nach einem der Ansprüche 1 bis 6 gerichtet sind, festzustellen.

23. Verfahren zur Erfassung von Antikörpern, die auf ein Protein nach einem der Ansprüche 1 bis 6 gerichtet sind, in einer Körperflüssigkeit aus einem Tier, wobei
(a) eine Mikortiterplatte mit einem Protein nach einem der Ansprüche 1 bis 6 beschichtet wird,
(b) die Mikrotiterplatte mit der besagten Körperflüssigkeit inkubiert wird,
(c) die Mikrotiterplatte mit einem markierten Konjugat inkubiert wird, und
(d) eine Farbreaktion initiiert wird, um einen Komplex des Antikörpers des Konjugats und Antikörper aus der Körperflüssigkeit, die auf ein Protein nach einem der Ansprüche 1 bis 6 gerichtet sind, festzustellen.

24. Verfahren zur Feststellung von Mycoplasma mycoides subsp. mycoides SC, wobei
(a) ein Paar von PCR-Primern für die Feststellung einer DNS nach der Sequenz ID Nr. 24 eingesetzt wird in einer PCR-Reaktion mit einer zu testenden Probe, und
(b) nach der PCR-Reaktion die Präsenz des PCR-Produkts, welches mit dem Paar von PCR-Primern erwartet wird, überprüft wird.

25. Verfahren nach Anspruch 24, bei dem das Paar von PCR-Primern ausgewählt wird aus einer Gruppe, umfassend die Primer der Sequenz ID Nr. 22 und Sequenz ID Nr. 23.

26. Monoklonaler Antikörper, der für ein Protein nach einem der Ansprüche 1 bis 6 spezifisch ist.

27. Diagnosekit mit einem Protein nach einem der Ansprüche 1 bis 6 und/oder mindestens einem Antikörper nach Anspruch 26 in einem geeignetem Behälter.

28. Impfstoff umfassend ein Protein nach einem der Ansprüche 1 bis 6 oder eine DNS nach einem der Ansprüche 7 oder 8 gegen die Infektion von Mycoplasma mycoides subsp. mycoides SC.

## Revendications

1. Protéine isolée de *Mycoplasma mycoides* subsp. *mycoides* à petites colonies (SC) comprenant la séquence d'acides aminés montrée dans SEQ ID NO: 25 ou des parties antigéniques de celle-ci.

2. Protéine isolée de précurseur LppQ de *Mycoplasma mycoides* subsp. *mycoides* SC, **caractérisée par** la séquence d'acides aminés de SEQ ID NO: 25.

3. Protéine isolée selon la revendication 1, dans laquelle les parties sont **caractérisées par** la séquence d'acides aminés montrée dans SEQ ID NO: 27 ou dans SEQ ID NO: 29.

4. Protéine LppQ mature isolée de *Mycoplasma mycoides* subsp. *mycoides* SC, **caractérisée par** la séquence d'acides aminés montrée dans SEQ IID NO: 27.

5. Partie N-terminale de protéine LppQ mature de *Mycoplasma mycoides* subsp. *mycoides* SC, **caractérisée par** la séquence d'acides aminés montrée dans SEQ ID NO: 29.

6. Protéine isolée selon l'une quelconque des revendications 1 à 5, dans laquelle la protéine est une lipoprotéine.

7. ADN codant pour une protéine selon l'une quelconque des revendications 1 à 6.

8. ADN selon la revendication 7, **caractérisé par** la séquence d'ADN montrée dans SEQ ID NO: 24, dans SEQ ID NO: 26 ou dans SEQ ID NO: 28.

9. Utilisation d'une protéine selon l'une quelconque des revendications 1 à 6, dans un procédé de diagnostic pour la détection directe ou indirecte de *Mycoplasma mycoides* subsp. *mycoides* SC.

10. Utilisation selon la revendication 9, dans laquelle le procédé de diagnostic est un procédé immunologique et est choisi dans un groupe comprenant les immunotransferts, les essais sérologiques et les essais ELISA.

11. Utilisation d'une protéine selon l'une quelconque des revendications 1 à 6 ou d'un ADN selon l'une quelconque des revendications 7 ou 8, pour la préparation d'un vaccin contre une infection provoquée par *Mycoplasma mycoides* subsp. *mycoides* SC.

12. Utilisation d'un ADN selon l'une quelconque des revendications 7 ou 8, pour la préparation de cellules vivantes, atténuées ou inactivées de *Mycoplasma mycoides* subsp. *mycoides* SC à utiliser comme vaccin marqué, où ces cellules sont dépourvues de l'aptitude à synthétiser la protéine LppQ.

13. Utilisation d'un ADN selon l'une quelconque des revendications 7 ou 8, dans un procédé de détection de *Mycoplasma mycoides* subsp. *mycoides* SC.

14. Utilisation selon la revendication 13, dans laquelle le procédé de détection est choisi dans un groupe comprenant les procédés de PCR et d'hybridation.

15. Utilisation d'un ADN selon l'une quelconque des revendications 7 ou 8, pour l'expression d'une protéine selon l'une quelconque des revendications 1 à 6.

16. Vecteur comprenant un ADN selon l'une quelconque des revendications 7 ou 8.

17. Vecteur selon la revendication 16, **caractérisé en ce que** ledit vecteur est le vecteur pJFFmaLP48-MuHisl tel que déposé à l'European Collection of Cell Cultures, Salisbury, Wiltshire SP40JG.UK sous le numéro de dépôt 99081025.

18. Cellule hôte comprenant un vecteur selon la revendication 16 ou 17, ou un ADN selon l'une quelconque des revendications 7 ou 8.

19. Procédé de préparation d'une protéine selon l'une quelconque des revendications 1 à 6, dans lequel
(a) un vecteur selon la revendication 16 ou 17 est introduit dans une cellule hôte convenable ;
(b) la cellule hôte est cultivée dans des conditions qui permettent l'expression d'une protéine selon l'une quelconque des revendications 1 à 6 ; et
(c) la protéine de l'étape (b) est isolée des cellules hôtes ou du surnageant.

20. Procédé de préparation d'un ADN **caractérisé par** la séquence d'ADN de SEQ ID NO: 25, dans lequel :
(a) une bibliothèque d'ADN, d'ADN génomique de *Mycoplasma mycoides* subsp. *mycoides* SC est clonée dans un phage convenable ;
(b) le phage de l'étape (a) est utilisé pour infecter des cellules hôtes convenables ;
(c) les cellules hôtes sont criblées avec des sérums provenant d'un mammifère infecté par *Mycoplasma mycoides* subsp. *mycoides* SC;
(d) des clones positifs sont choisis et purifiés ; et
(e) l'ADN génomique du phage de l'étape (d) est isolé.

21. Procédé de préparation d'un ADN, **caractérisé par** la séquence d'ADN de SEQ ID NO: 26 ou de SEQ ID NO: 28, dans lequel :
(a) un ADN **caractérisé par le fait qu'**il contient la séquence d'ADN de SEQ ID NO: 24 et que des ensembles d'amorces selon les SEQ ID NO: 1 à 4 et 6 à 21 sont appliqués dans une PCR avec extension de recouvrement (OE-PCR) pour donner un premier produit de PCR ;
(b) le produit de PCR de la OE-PCR de l'étape (a) est appliqué dans une ou plusieurs OE-PCR selon ce qui est requis par le nombre de mutations recherchées pour donner un produit final de PCR ; et
(c) le produit final de PCR est isolé.

22. Procédé de détection d'anticorps dirigés contre une protéine selon l'une quelconque des revendications 1 à 6 dans un fluide corporel provenant d'un animal, dans lequel :
(a) une protéine selon l'une quelconque des revendications 1 à 6 est transférée sur une membrane convenable ;
(b) la membrane est incubée avec ledit fluide corporel ;
(c) la membrane est incubée avec un conjugué marqué ; et
(d) une réaction de coloration est initiée afin de détecter un complexe de l'anticorps du conjugué et d'anticorps provenant du fluide corporel dirigés contre une protéine selon l'une quelconque des revendications 1 à 6.

23. Procédé de détection d'anticorps dirigés contre une protéine selon l'une quelconque des revendications 1 à 6 dans un fluide corporel provenant d'un animal, dans lequel :
(a) une plaque de microtitrage est revêtue d'une protéine selon l'une quelconque des revendications 1 à 6 ;
(b) la plaque de microtitrage est incubée avec ledit fluide corporel ;
(c) la plaque de microtitrage est incubée avec un conjugué marqué ; et
(d) une réaction de coloration est initiée afin de détecter un complexe de l'anticorps du conjugué et d'anticorps provenant du fluide corporel dirigés contre une protéine selon l'une quelconque des revendications 1 à 6.

24. Procédé de détection de *Mycoplasma mycoides* subsp. *mycoides* SC, dans lequel
(a) une paire d'amorces de PCR pour la détection d'un ADN selon SEQ ID NO: 24 est utilisée dans une réaction de PCR avec un échantillon à tester ; et
(b) après la réaction de PCR, la présence du produit de PCR attendu avec la paire d'amorces de PCR est vérifiée.

25. Procédé selon la revendication 24, dans lequel la paire d'amorces de PCR est choisie dans un groupe comprenant les amorces de SEQ ID NO: 22 et SEQ ID NO: 23.

26. Anticorps monoclonal spécifique pour une protéine selon l'une quelconque des revendications 1 à 6.

27. Kit de diagnostic contenant une protéine selon l'une quelconque des revendications 1 à 6 et/ou au moins un anticorps selon la revendication 26 dans un récipient convenable.

28. Vaccin comprenant une protéine selon l'une quelconque des revendications 1 à 6 ou un ADN selon l'une quelconque des revendications 7 ou 8 contre une infection par *Mycoplasma mycoides* subsp. *mycoides* SC.


### SEQUENCE LISTING

<110> AKZO Nobel N.V.
<120> Antigenic protein LppQ of Mycoplasma mycoides subsp. mycoides SC, its preparation and use
<130> OL1-99PRIO
<160> 31
<170> PatentIn Ver. 2.1
<210> 1
   <211> 33
   <212> DNA
   <213> Mycoplasma mycoides
<221> primer_bind
   <222> (1)..(33)
<400> 1
   cgccatatgc cattagttgt tgtttcatgt aaa 33
<210> 2
   <211> 25
   <212> DNA
   <213> Mycoplasma mycoides
<221> primer_bind
   <222> (1)..(25)
<400> 2
   tcttgttttt gccactgatt ttttg 25
<210> 3
   <211> 25
   <212> DNA
   <213> Mycoplasma mycoides
<221> primer_bind
   <222> (1)..(25)
<400> 3
   caaaaaatga gtggcaaaaa caaga 25
<210> 4
   <211> 34
   <212> DNA
   <213> Mycoplasma mycoides
<221> primer_bind
   <222> (1)..(34)
<400> 4
   ccggatcccc aatttgataa aacttgatta aagt 34
<210> 5
   <211> 37
   <212> DNA
   <213> Mycoplasma mycoides
<221> primer_bind
   <222> (1)..(37)
<400> 5
   aacgaattca gttttatcaa attggaatac agaaaat 37
<210> 6
   <211> 28
   <212> DNA
   <213> Mycoplasma mycoides
<221> primer_bind
   <222> (1)..(28)
<400> 6
   catttgctgt tttccagctc gataaatc 28
<210> 7
   <211> 30
   <212> DNA
   <213> Mycoplasma mycoides
<221> primer_bind
   <222> (1)..(30)
<400> 7
   gtgatttatc gagctggaaa acagcaaatg 30
<210> 8
   <211> 30
   <212> DNA
   <213> Mycoplasma mycoides
<221> primer_bind
   <222> (1)..(30)
<400> 8
   cattacttac attccaactc gaaatatctt 30
<210> 9
   <211> 30
   <212> DNA
   <213> Mycoplasma mycoides
<221> primer_bind
   <222> (1)..(30)
<400> 9
   aagatatttc gagttggaat gtaagtaatg 30
<210> 10
   <211> 30
   <212> DNA
   <213> Mycoplasma mycoides
<221> primer_bind
   <222> (1)..(30)
<400> 10
   atatttttta gattccaatc tgaaagtgat 30
<210> 11
   <211> 30
   <212> DNA
   <213> Mycoplasma mycoides
<221> primer_bind
   <222> (1)..(30)
<400> 11
   atcactttca gattggaatc taaaaaatat 30
<210> 12
   <211> 30
   <212> DNA
   <213> Mycoplasma mycoides
<221> primer_bind
   <222> (1)..(30)
<400> 12
   cttttgatac atcccaatct aaagacttat 30
<210> 13
   <211> 30
   <212> DNA
   <213> Mycoplasma mycoides
<221> primer bind
   <222> (1)..(30)
<400> 13
   ataagtcttt agattgggat gtatcaaaag 30
<210> 14
   <211> 29
   <212> DNA
   <213> Mycoplasma mycoides
<221> primer_bind
   <222> (1)..(29)
<400> 14
   tttgaaacat tccaattagt tatattttg 29
<210> 15
   <211> 31
   <212> DNA
   <213> Mycoplasma mycoides
<221> primer_bind
   <222> (1)..(31)
<400> 15
   tcaaaatata actaattgga atgtttcaaa t 31
<210> 16
   <211> 30
   <212> DNA
   <213> Mycoplasma mycoides
<221> primer_bind
   <222> (1)..(30)
<400> 16
   acattatcaa ctctccaatt ttttatatct 30
<210> 17
   <211> 31
   <212> DNA
   <213> Mycoplasma mycoides
<221> primer_bind
   <222> (1)..(31)
<400> 17
   agatataaaa aattggagag ttgataatgt a 31
<210> 18
   <211> 38
   <212> DNA
   <213> Mycoplasma mycoides
<221> primer_bind
   <222> (1)..(38)
<400> 18
   ccggatccta cattttttac attccaactt gaaatatc 38
<210> 19
   <211> 30
   <212> DNA
   <213> Mycoplasma mycoides
<221> primer_bind
   <222> (1)..(30)
<400> 19
   gttttatcaa attggaatac agaaaatgta 30
<210> 20
   <211> 30
   <212> DNA
   <213> Mycoplasma mycoides
<221> primer_bind
   <222> (1)..(30)
<400> 20
   tacattttct gtattccaat ttgataaaac 30
<210> 21
   <211> 33
   <212> DNA
   <213> Mycoplasma mycoides
<221> primer_bind
   <222> (1)..(33)
<400> 21
   gcagaattcc cattagttgt tgtttcatgt aaa 33
<210> 22
   <211> 19
   <212> DNA
   <213> Mycoplasma mycoides
<221> primer_bind
   <222> (1)..(19)
<400> 22
   actctgagag ggaataatg 19
<210> 23
   <211> 18
   <212> DNA
   <213> Mycoplasma mycoides
<221> primer_bind
   <222> (1)..(18)
<400> 23
   caaccaaagg catcaatg 18
<210> 24
   <211> 1335
   <212> DNA
   <213> Mycoplasma mycoides
<221> gene
   <222> (1)..(1335)
<400> 24
   atgaaaaata aacatatcag tctactagca aaacttgaag ttttattttc tgttacatca 60
   cttccattag ttgttgtttc atgtaaaact tcaaatttta ataacaataa acctaataat 120
   aatcaacaaa aaaaagagca agtaagtaaa attgatattt caagttttaa agataaaatt 180
   gaaccaaaaa atgagtgaca aaaacaagat gttttacaag cattactaaa aataaaaggg 240
   ctagataaat taactcaaaa tgattttaat tttaatatta agaaagctaa tttattacgt 300
   aatggacaat tgataattaa atctaaagac gattctaaaa ttataaaagg tgaactaagt 360
   ttagaaatta aaaaactaaa tagagttaaa aaagttgaaa caaaatataa tgatactaga 420
   actgaggttt tagtaattgg ttatgatgaa aatggaaaaa tttctgggtt tgctcaaact 480
   gttaaaaaag ttcctgaaaa actaccagaa gaaattatta gtctagagcg tgctttttta 540
   aaaaataatt ctgataaaat tgaaaatctt gataaatggg acacatctaa tatagtaagt 600
   atgtcttcaa tgtttcaaca agctagaaac tttaatcaag ttttatcaaa ttgaaataca 660
   gaaaatgtaa ctgatatgaa ttatatgttt gatggtgcaa ctaaattcaa tagtgattta 720
   tcgagctgaa aaacagcaaa tgtaaaaact atgagaagta tgtttagtga cacaaaacaa 780
   tttaatcaag atatttcgag ttgaaatgta agtaatgtta aaaatatgaa aaatatgttt 840
   tatcgagctg aaaaatttaa taaatcactt tcagattgaa atctaaaaaa tatacaagaa 900
   ctagaccata tgttttttgg tgcttctgaa tttaatagcg atatcttcaa actaaaaaat 960
   aatctagtaa ctgatatgcg ttatatgttt tttcaagcta aaaaatttaa taagtcttta 1020
   gattgagatg tatcaaaagt agttaatatg gatagtatgt ttaatggtgc tcatgatttt 1080
   aatcaaaata taactaattg aaatgtttca aatgttaaaa ctatgagaag tatgtttagt 1140
   gacacaaaac aatttaatca agatataaaa aattgaagag ttgataatgt aactgatatg 1200
   gaccgtatgt ttcaaaatgc actaagtttt aataaagata tttcaagttg aaatgtaaaa 1260
   aatgtaaaat catatgatgc ctttggttga cacataaaaa aagaatttaa acctttattt 1320
   gaaaaaaata ataag                                                   1335
<210> 25
   <211> 445
   <212> PPT
   <213> Mycoplasma mycoides
<221> PEPTIDE
   <222> (1)..(445)
<400> 25
   Met Lys Asn Lys His Ile Ser Leu Leu Ala Lys Leu Glu Val Leu Phe
   1               5                   10                  15
   Ser Val Thr Ser Leu Pro Leu Val Val Val Ser Cys Lys Thr Ser Asn
               20                  25                  30
   Phe Asn Asn Asn Lys Pro Asn Asn Asn Gln Gln Lys Lys Glu Gln Val
           35                  40                  45
   Ser Lys Ile Asp Ile Ser Ser Phe Lys Asp Lys Ile Glu Pro Lys Asn
       50                  55                  60
   Glu Trp Gln Lys Gln Asp Val Leu Gln Ala Leu Leu Lys Ile Lys Gly
   65                  70                  75                  80
   Leu Asp Lys Leu Thr Gln Asn Asp Phe Asn Phe Asn Ile Lys Lys Ala
                   85                  90                  95
   Asn Leu Leu Arg Asn Gly Gln Leu Ile Ile Lys Ser Lys Asp Asp Ser
               100                 105                 110
   Lys Ile Ile Lys Gly Glu Leu Ser Leu Glu Ile Lys Lys Leu Asn Arg
           115                 120                 125
   Val Lys Lys Val Glu Thr Lys Tyr Asn Asp Thr Arg Thr Glu Val Leu
       130                 135                 140
   Val Ile Gly Tyr Asp Glu Asn Gly Lys Ile Ser Gly Phe Ala Gln Thr
   145                 150                 155                 160
   Val Lys Lys Val Pro Glu Lys Leu Pro Glu Glu Ile Ile Ser Leu Glu
                   165                 170                 175
   Arg Ala Phe Leu Lys Asn Asn Ser Asp Lys Ile Glu Asn Leu Asp Lys
               180                 185                 190
   Trp Asp Thr Ser Asn Ile Val Ser Met Ser Ser Met Phe Gln Gln Ala
           195                 200                 205
   Arg Asn Phe Asn Gln Val Leu Ser Asn Trp Asn Thr Glu Asn Val Thr
       210                 215                 220
   Asp Met Asn Tyr Met Phe Asp Gly Ala Thr Lys Phe Asn Ser Asp Leu
   225                 230                 235                 240
   Ser Ser Trp Lys Thr Ala Asn Val Lys Thr Met Arg Ser Met Phe Ser
                   245                 250                 255
   Asp Thr Lys Gln Phe Asn Gln Asp Ile Ser Ser Trp Asn Val Ser Asn
               260                 265                 270
   Val Lys Asn Met Lys Asn Met Phe Tyr Arg Ala Glu Lys Phe Asn Lys
           275                 280                 285
   Ser Leu Ser Asp Trp Asn Leu Lys Asn Ile Gln Glu Leu Asp His Met
       290                 295                 300
   Phe Phe Gly Ala Ser Glu Phe Asn Ser Asp Ile Phe Lys Leu Lys Asn
   305                 310                 315                 320
   Asn Leu Val Thr Asp Met Arg Tyr Met Phe Phe Gln Ala Lys Lys Phe
                   325                 330                 335
   Asn Lys Ser Leu Asp Trp Asp Val Ser Lys Val Val Asn Met Asp Ser
               340                 345                 350
   Met Phe Asn Gly Ala His Asp Phe Asn Gln Asn Ile Thr Asn Trp Asn
           355                 360                 365
   Val Ser Asn Val Lys Thr Met Arg Ser Met Phe Ser Asp Thr Lys Gln
       370                 375                 380
   Phe Asn Gln Asp Ile Lys Asn Trp Arg Val Asp Asn Val Thr Asp Met
   385                 390                 395                 400
   Asp Arg Met Phe Gln Asn Ala Leu Ser Phe Asn Lys Asp Ile Ser Ser
                   405                 410                 415
   Trp Asn Val Lys Asn Val Lys Ser Tyr Asp Ala Phe Gly Trp His Ile
               420                 425                 430
   Lys Lys Glu Phe Lys Pro Leu Phe Glu Lys Asn Asn Lys
           435                 440                 445
<210> 26
   <211> 1185
   <212> DNA
   <213> Mycoplasma mycoides
<221> gene
   <222> (1)..(1185)
<400> 26
   tgtaaaactt caaattttaa taacaataaa cctaataata atcaacaaaa aaaagagcaa 60
   gtaagtaaaa ttgatatttc aagttttaaa gataaaattg aaccaaaaaa tgagtggcaa 120
   aaacaagatg ttttacaagc attactaaaa ataaaagggc tagataaatt aactcaaaat 180
   gattttaatt ttaatattaa gaaagctaat ttattacgta atggacaatt gataattaaa 240
   tctaaagacg attctaaaat tataaaaggt gaactaagtt tagaaattaa aaaactaaat 300
   agagttaaaa aagttgaaac aaaatataat gatactagaa ctgaggtttt agtaattggt 360
   tatgatgaaa atggaaaaat ttctgggttt gctcaaactg ttaaaaaagt tcctgaaaaa 420
   ctaccagaag aaattattag tctagagcgt gcttttttaa aaaataattc tgataaaatt 480
   gaaaatcttg ataaatggga cacatctaat atagtaagta tgtcttcaat gtttcaacaa 540
   gctagaaact ttaatcaagt tttatcaaat tggaatacag aaaatgtaac tgatatgaat 600
   tatatgtttg atggtgcaac taaattcaat agtgatttat cgagctggaa aacagcaaat 660
   gtaaaaacta tgagaagtat gtttagtgac acaaaacaat ttaatcaaga tatttcgagt 720
   tggaatgtaa gtaatgttaa aaatatgaaa aatatgtttt atcgagctga aaaatttaat 780
   aaatcacttt cagattggaa tctaaaaaat atacaagaac tagaccatat gttttttggt 840
   gcttctgaat ttaatagcga tatcttcaaa ctaaaaaata atctagtaac tgatatgcgt 900
   tatatgtttt ttcaagctaa aaaatttaat aagtctttag attgggatgt atcaaaagta 960
   gttaatatgg atagtatgtt taatggtgct catgatttta atcaaaatat aactaattgg 1020
   aatgtttcaa atgttaaaac tatgagaagt atgtttagtg acacaaaaca atttaatcaa 1080
   gatataaaaa attggagagt tgataatgta actgatatgg accgtatgtt tcaaaatgca 1140
   ctaagtttta ataaagatat ttcaagttgg aatgtaaaaa atgta                 1185
<210> 27
   <211> 395
   <212> PRT
   <213> Mycoplasma mycoides
<221> PEPTIDE
   <222> (1)..(395)
<400> 27
   Cys Lys Thr Ser Asn Phe Asn Asn Asn Lys Pro Asn Asn Asn Gln Gln
   1               5                   10                  15
   Lys Lys Glu Gln Val Ser Lys Ile Asp Ile Ser Ser Phe Lys Asp Lys
               20                  25                  30
   Ile Glu Pro Lys Asn Glu Trp Gln Lys Gln Asp Val Leu Gln Ala Leu
           35                  40                  45
   Leu Lys Ile Lys Gly Leu Asp Lys Leu Thr Gln Asn Asp Phe Asn Phe
       50                  55                  60
   Asn Ile Lys Lys Ala Asn Leu Leu Arg Asn Gly Gln Leu Ile Ile Lys
   65                  70                  75                  80
   Ser Lys Asp Asp Ser Lys Ile Ile Lys Gly Glu Leu Ser Leu Glu Ile
                   85                  90                  95
   Lys Lys Leu Asn Arg Val Lys Lys Val Glu Thr Lys Tyr Asn Asp Thr
               100                 105                 110
   Arg Thr Glu Val Leu Val Ile Gly Tyr Asp Glu Asn Gly Lys Ile Ser
           115                 120                 125
   Gly Phe Ala Gln Thr Val Lys Lys Val Pro Glu Lys Leu Pro Glu Glu
       130                 135                 140
   Ile Ile Ser Leu Glu Arg Ala Phe Leu Lys Asn Asn Ser Asp Lys Ile
   145 150 155 160
   Glu Asn Leu Asp Lys Trp Asp Thr Ser Asn Ile Val Ser Met Ser Ser
   165 170 175
   Met Phe Gln Gln Ala Arg Asn Phe Asn Gln Val Leu Ser Asn Trp Asn
   180 185 190
   Thr Glu Asn Val Thr Asp Met Asn Tyr Met Phe Asp Gly Ala Thr Lys
   195 200 205
   Phe Asn Ser Asp Leu Ser Ser Trp Lys Thr Ala Asn Val Lys Thr Met
   210 215 220
   Arg Ser Met Phe Ser Asp Thr Lys Gln Phe Asn Gln Asp Ile Ser Ser
   225 230 235 240
   Trp Asn Val Ser Asn Val Lys Asn Met Lys Asn Met Phe Tyr Arg Ala
   245 250 255
   Glu Lys Phe Asn Lys Ser Leu Ser Asp Trp Asn Leu Lys Asn Ile Gln
   260 265 270
   Glu Leu Asp His Met Phe Phe Gly Ala Ser Glu Phe Asn Ser Asp Ile
   275 280 285
   Phe Lys Leu Lys Asn Asn Leu Val Thr Asp Met Arg Tyr Met Phe Phe
   290 295 300
   Gln Ala Lys Lys Phe Asn Lys Ser Leu Asp Trp Asp Val Ser Lys Val
   305 310 315 320
   Val Asn Met Asp Ser Met Phe Asn Gly Ala His Asp Phe Asn Gln Asn
   325 330 335
   Ile Thr Asn Trp Asn Val Ser Asn Val Lys Thr Met Arg Ser Met Phe
   340 345 350
   Ser Asp Thr Lys Gln Phe Asn Gln Asp Ile Lys Asn Trp Arg Val Asp
   355 360 365
   Asn Val Thr Asp Met Asp Arg Met Phe Gln Asn Ala Leu Ser Phe Asn
   370 375 380
   Lys Asp Ile Ser Ser Trp Asn Val Lys Asn Val
   385 390 395
<210> 28
   <211> 588
   <212> DNA
   <213> Mycoplasma mycoides
<221> gene
   <222> (1)..(588)
<400> 28
   tgtaaaactt caaattttaa taacaataaa cctaataata atcaacaaaa aaaagagcaa 60
   gtaagtaaaa ttgatatttc aagttttaaa gataaaattg aaccaaaaaa tgagtggcaa 120
   aaacaagatg ttttacaagc attactaaaa ataaaagggc tagataaatt aactcaaaat 180
   gattttaatt ttaatattaa gaaagctaat ttattacgta atggacaatt gataattaaa 240
   tctaaagacg attctaaaat tataaaaggt gaactaagtt tagaaattaa aaaactaaat 300
   agagttaaaa aagttgaaac aaaatataat gatactagaa ctgaggtttt agtaattggt 360
   tatgatgaaa atggaaaaat ttctgggttt gctcaaactg ttaaaaaagt tcctgaaaaa 420
   ctaccagaag aaattattag tctagagcgt gcttttttaa aaaataattc tgataaaatt 480
   gaaaatcttg ataaatggga cacatctaat atagtaagta tgtcttcaat gtttcaacaa 540
   gctagaaact ttaatcaagt tttatcaaat tggaatacag aaaatgta              588
<210> 29
   <211> 196
   <212> PRT
   <213> Mycoplasma mycoides
<221> PEPTIDE
   <222> (1)..(196)
<210> 30
   <211> 627
   <212> DNA
   <213> Mycoplasma mycoides
<221> gene
   <222> (1)..(627)
<400> 30
   gttttatcaa attggaatac agaaaatgta actgatatga attatatgtt tgatggtgca 60
   actaaattca atagtgattt atcgagctgg aaaacagcaa atgtaaaaac tatgagaagt 120
   atgtttagtg acacaaaaca atttaatcaa gatatttcga gttggaatgt aagtaatgtt 180
   aaaaatatga aaaatatgtt ttatcgagct gaaaaattta ataaatcact ttcagattgg 240
   aatctaaaaa atatacaaga actagaccat atgttttttg gtgcttctga atttaatagc 300
   gatatcttca aactaaaaaa taatctagta actgatatgc gttatatgtt ttttcaagct 360
   aaaaaattta ataagtcttt agattgggat gtatcaaaag tagttaatat ggatagtatg 420
   tttaatggtg ctcatgattt taatcaaaat ataactaatt ggaatgtttc aaatgttaaa 480
   actatgagaa gtatgtttag tgacacaaaa caatttaatc aagatataaa aaattggaga 540
   gttgataatg taactgatat ggaccgtatg tttcaaaatg cactaagttt taataaagat 600
   atttcaagtt ggaatgtaaa aaatgta 627
<210> 31
   <211> 209
   <212> PRT
   <213> Mycoplasma mycoides
<221> PEPTIDE
   <222> (1)..(209)
   <400> 31
   Val Leu Ser Asn Trp Asn Thr Glu Asn Val Thr Asp Met Asn Tyr Met
   1               5                   10                  15
   Phe Asp Gly Ala Thr Lys Phe Asn Ser Asp Leu Ser Ser Trp Lys Thr
               20                  25                  30
   Ala Asn Val Lys Thr Met Arg Ser Met Phe Ser Asp Thr Lys Gln Phe
           35                  40                  45
   Asn Gln Asp Ile Ser Ser Trp Asn Val Ser Asn Val Lys Asn Met Lys
       50                  55                  60
   Asn Met Phe Tyr Arg Ala Glu Lys Phe Asn Lys Ser Leu Ser Asp Trp
   65                  70                  75                  80
   Asn Leu Lys Asn Ile Gln Glu Leu Asp His Met Phe Phe Gly Ala Ser
                   85                  90                  95
   Glu Phe Asn Ser Asp Ile Phe Lys Leu Lys Asn Asn Leu Val Thr Asp
               100                 105                 110
   Met Arg Tyr Met Phe Phe Gln Ala Lys Lys Phe Asn Lys Ser Leu Asp
           115                 120                 125
   Trp Asp Val Ser Lys Val Val Asn Met Asp Ser Met Phe Asn Gly Ala
       130                 135                 140
   His Asp Phe Asn Gln Asn Ile Thr Asn Trp Asn Val Ser Asn Val Lys
   145                 150                 155                 160
   Thr Met Arg Ser Met Phe Ser Asp Thr Lys Gln Phe Asn Gln Asp Ile
                   165                 170                 175
   Lys Asn Trp Arg Val Asp Asn Val Thr Asp Met Asp Arg Met Phe Gln
               180                 185                 190
   Asn Ala Leu Ser Phe Asn Lys Asp Ile Ser Ser Trp Asn Val Lys Asn
           195                 200                 205
   Val